# EUROPEAN PATENT APPLICATION

(11) **EP 1 637 597 A1**
(43) Date of publication of application: **22.03.2006**
(21) Application number: 04735817.1
(22) Date of filing: 02.06.2004
(51) Int. Cl.: C12N 15/00, C07K 14/47, C12N 5/00, C12Q 1/68

(54) **METHOD OF NUCLEIC ACID INFUSION**

(30) Priority: 06.06.2003 JP 2003161498
(71) Applicant: Dainippon Sumitomo Pharma Co., Ltd., Osaka 541-8524 (JP); Sumitomo Chemical Company, Limited, Tokyo 104-8260 (JP)
(72) Inventor: AOKI, Mikio, SUMITOMO PHARMACEUTICALS CO., LTD., Osaka-shi, Osaka 554-0022 (JP)
(74) Representative: Duckworth, Timothy John
(86) International application number: PCT/JP2004/008020
(87) International publication number: WO 2004/108921

(57) **Abstract**

A method of nucleic acid infusion, comprising the step (a) of bringing a nucleic acid, a hypertonic solution and cells into contact with each other and the step (b) of lowering the osmotic pressure of the hypertonic solution after the step (a). There is further provided a reagent for nucleic acid infusion, comprising as an ingredient at least one substance belonging to the category of oligosaccharide or polyhydric alcohol.

SEQUENCE LISTING

<110>  Sumitomo Pharmaceuticals Co., Ltd.
       Sumitomo Chemical Co., Ltd.

<120>  Nucleic Acid Delivery System

<130>  533712

<150>  JP2003-161498
<151>  2003-06-06

<160>  18

<170>  PatentIn version 3.2

<210>  1
<211>  20
<212>  DNA
<213>  Artificial

<220>
<223>  Synthetic Oligonucleotide

<400>  1
ggaacttccc taaagggagg                                                    20

<210>  2
<211>  21
<212>  RNA
<213>  Artificial

<220>
<223>  Synthetic Oligonucleotide

<400>  2
auccacaagu caaucucucu u                                                  21

<210>  3
<211>  21
<212>  RNA

<213>  Artificial

<220>
<223>  Synthetic Oligonucleotide

<400>  3
gagagauuga cuuguggauu u                                                21


<210>  4
<211>  21
<212>  RNA
<213>  Artificial

<220>
<223>  Synthetic Oligonucleotide

<400>  4
caagcugacc cugaaguuca u                                                21


<210>  5
<211>  21
<212>  RNA
<213>  Artificial

<220>
<223>  Synthetic Oligonucleotide

<400>  5
gaacuucagg gucagcuugu u                                                21


<210>  6
<211>  21
<212>  RNA
<213>  Artificial

<220>
<223>  Synthetic Oligonucleotide

<400>  6
ccacgagaaa uaugacaacu c                                                21

<210> 7
<211> 21
<212> RNA
<213> Artificial

<220>
<223> Synthetic Oligonucleotide

<400> 7
guugucauau uucucguggu u 21

<210> 8
<211> 25
<212> DNA
<213> Artificial

<220>
<223> Synthetic Oligonucleotide

<400> 8
cctcttacct cagttacaat ttata 25

<210> 9
<211> 21
<212> RNA
<213> Artificial

<220>
<223> Synthetic Oligonucleotide

<400> 9
gucuaaaguc gauccgcgac a 21

<210> 10
<211> 21
<212> RNA
<213> Artificial

<220>
<223>  Synthetic Oligonucleotide

<400>  10
ucgcggaucg acuuuagacu u                                                21


<210>  11
<211>  18
<212>  DNA
<213>  Artificial

<220>
<223>  PCR Primer

<400>  11
ggatgatgcc tccctggc                                                    18


<210>  12
<211>  20
<212>  DNA
<213>  Artificial

<220>
<223>  PCR Primer

<400>  12
gggattcaag cttcctggtg                                                  20


<210>  13
<211>  18
<212>  DNA
<213>  Artificial

<220>
<223>  PCR Primer

<400>  13
aactcggccc ccaacact                                                    18

<210> 14
<211> 22
<212> DNA
<213> Artificial

<220>
<223> PCR Primer

<400> 14
taggcccctc ctgttattat gg 22


<210> 15
<211> 19
<212> DNA
<213> Artificial

<220>
<223> PCR Primer

<400> 15
tcaccaccat ggagaaggc 19


<210> 16
<211> 20
<212> DNA
<213> Artificial

<220>
<223> PCR Primer

<400> 16
ctaagcagtt ggtggtgcag 20


<210> 17
<211> 19
<212> DNA
<213> Artificial

<220>
<223> PCR Primer

<400>  17
gtcaacccca ccgtgttct                                                    19

<210>  18
<211>  20
<212>  DNA
<213>  Artificial

<220>
<223>  PCR Primer

<400>  18
gaggtacctg ctgtctttgg                                                   20

## Description

### TECHNICAL FIELD

The present invention relates to a novel method of nucleic acid infusion. More particularly, the present invention relates to a novel method of nucleic acid infusion which utilizes difference in osmotic pressures.

### BACKGROUND ART

The efficient infusion of oligonucleotide such as antisense nucleic acids and small interfering RNA (siRNA) into cells is quite important for gene functional analysis that uses these molecules. Conventional methods of gene infusion include: a method of infusing into a cell a complex formed through an ionic bond between a functional molecule and cell membrane negatively charged and a carrier positively charged such as a cationic liposome (Felgner et al., Proc. Natl. Acad. Sci. USA, 84, 7413 (1987); and Invitrogen, FOCUS, Vol. 21, No. 3 (1999)) or polyethylenimine (Boussif et al., Proc. Natl. Acad. Sci. USA, 92, 7297 (1995)); a method of infusing a fusion molecule formed through a covalent bond between cell-permeable peptide and oligonucleotide (Thoren et al., FEBS Letters, 482, 265 (2000); and Nagahara et al., Nature medicine, 4, 1449 (1998)); and a method of infusing a functional molecule into a hole that is transiently made on a cell by the application of electric pulses (Neumann et al., EMBO J, 1, 841 (1982)).

The method of infusion that uses a complex with a positively charged carrier generally has the favorable efficiency of infusion into adherent culture cell lines. However, the method uses uptake mechanisms intrinsic to cells in the cellular uptake of the oligonucleotide-carrier complex from the outside to the inside and therefore has a difficulty with infusion into cells having low uptake activity. Moreover, the carrier itself has cytotoxicity and immunoadjuvant activity and is therefore difficult to infuse into floating cells such as lymphocytes and primary cultured cells. The method of infusing a fusion molecule formed through a covalent bond between cell-permeable peptide and oligonucleotide presents problems with the reaction efficiency of covalent bond formation and the complicated reaction and also presents problems in that the method observes not the action of the oligonucleotide alone but the overall action of the molecule formed through the covalent bond between the cell-permeable peptide and the functional molecule. Alternatively, the method of infusing a functional molecule into a hole that is transiently made on a cell by the application of electric pulses presents problems in that cells suffer severe damage due to the electric pulses, resulting in the disruption of most of the cells. Thus, each of the conventional methods of nucleic acid infusion has problems. Under these circumstances, there has been a demand for a novel method of nucleic acid infusion that is capable of efficiently infusing a nucleic acid, regardless of cell types.

### DISCLOSURE OF THE INVENTION

An object of the present invention is to provide a novel method of nucleic acid infusion. More particularly, an object of the present invention is to provide a novel method of nucleic acid infusion that utilizes osmotic pressure difference.

The present inventors have diligently studied for attaining the above-described objects. As a result, the present inventors have successfully developed a method of efficient nucleic acid infusion into a cell, which is independent of carriers, cell-permeable peptide and electric pulses. That is, the present invention allows the efficient infusion of a nucleic acid such as oligonucleotide into a cell by utilizing osmotic pressure difference between an extracellular fluid and an intracellular fluid to promote pinocytosis that is known as one of phenomena in which cells take up extracellular solutions.

The infusion of low molecular weight compounds and proteins into cells by use of pinocytosis has previously been reported (Craig Y Okada et al., Cell, 29, 33-41, 1982). However, recently, this technique has already been rendered obsolete and has not been reported to be used in nucleic acid infusion.

The present inventors have investigated a method of nucleic acid infusion that utilizes osmotic pressure difference. That is, the present inventors have diligently conducted studies on a method of nucleic acid infusion by (1) bringing a hypertonic solution containing a nucleic acid and cells into contact with each other to induce the cellular uptake of the nucleic acid by pinocytosis and (2) then lowering the osmotic pressure of the above-described hypertonic solution to induce the disruption of pinocytic vesicles in the cells and the release of the nucleic acid into the cells. As a result, surprisingly, the present inventors have found that the use of the method of the present invention allows nucleic acid infusion with higher efficiency than ever. In addition, the present inventors have found that the method of the present invention allows efficient oligonucleotide infusion into cell types that are hardly infused with a nucleic acid by conventional techniques. Since the method of the present invention does not employ adjuvants (such as liposomes and polyethylenimine) conventionally used, the method overcomes the problems of conventional methods such as cytotoxicity and immunoadjuvant activity. Therefore, the present invention makes it possible to efficiently infuse oligonucleotide into not only adherent cell lines but also primary cultured cells and lymphocytes that are highly sensitive to cytotoxicity.

The present invention has been completed on the basis of such findings.

Namely, the present invention is as follows:
(1) a method of nucleic acid infusion, comprising the following steps (a) and (b):
   (a) bringing a nucleic acid, a hypertonic solution and cells into contact with each other; and
   (b) lowering osmotic pressure of the hypertonic solution after the above-described step (a);
(2) the method of nucleic acid infusion according to the above-described (1), wherein the above-described step (b) comprises lowering the osmotic pressure by bringing a hypotonic solution and the cells into contact with each other;
(3) the method of nucleic acid infusion according to the above-described (1) or (2), wherein the nucleic acid is oligonucleotide;
(4) the method of nucleic acid infusion according to the above-described (3), wherein the oligonucleotide is single-stranded oligonucleotide, double-stranded oligonucleotide or an analog thereof;
(5) the method of nucleic acid infusion according to the above-described (3) or (4), wherein the oligonucleotide is deoxyribonucleotide (DNA), ribonucleotide (RNA), phosphorothioate oligodeoxynucleotide, a 2'-O-(2-methoxy)ethyl-modified nucleic acid (2'-MOE-modified nucleic acid), small interfering RNA (siRNA), a locked nucleic acid (LNA), a peptide nucleic acid (PNA) or morpholino antisense oligonucleotide;
(6) the method of nucleic acid infusion according to any of the above-described (1) to (5), wherein the hypertonic solution comprises at least one substance which is oligosaccharide or polyhydric alcohol;
(7) the method of nucleic acid infusion according to the above-described (6), wherein the oligosaccharide is disaccharide;
(8) the method of nucleic acid infusion according to the above-described (7), wherein the disaccharide is sucrose, maltose or lactose;
(9) the method of nucleic acid infusion according to the above-described (6), wherein the polyhydric alcohol is diol, triol, polyol or sugar alcohol;
(10) the method of nucleic acid infusion according to the above-described (9), wherein the diol is a glycol derivative;
(11) the method of nucleic acid infusion according to the above-described (9), wherein the triol is a glycerol derivative;
(12) the method of nucleic acid infusion according to the above-described (9), wherein the polyol is a polyglycol derivative;
(13) the method of nucleic acid infusion according to the above-described (9), wherein the sugar alcohol is monosaccharide alcohol or oligosaccharide alcohol;
(14) the method of nucleic acid infusion according to the above-described (13), wherein the oligosaccharide alcohol is disaccharide alcohol;
(15) the method of nucleic acid infusion according to the above-described (10), wherein the glycol derivative is ethylene glycol;
(16) the method of nucleic acid infusion according to the above-described (11), wherein the glycerol derivative is glycerol;
(17) the method of nucleic acid infusion according to the above-described (12), wherein the polyglycol derivative is polyethylene glycol;
(18) the method of nucleic acid infusion according to the above-described (17), wherein the polyethylene glycol has a molecular weight of 2000 or less;
(19) the method of nucleic acid infusion according to the above-described (18), wherein the polyethylene glycol is PEG200, PEG300, PEG400, PEG600, PEG1000, PEG1500, PEG1540 or PEG 2000;
(20) the method of nucleic acid infusion according to the above-described (13), wherein the monosaccharide alcohol is mannitol, sorbitol, xylitol or erythritol;
(21) the method of nucleic acid infusion according to the above-described (14), wherein the disaccharide alcohol is maltitol, lactitol or reduced palatinose;
(22) the method of nucleic acid infusion according to the above-described (6), wherein the hypertonic solution comprises (a) at least one substance which is oligosaccharide or sugar alcohol and (b) at least one substance which is diol, triol or polyol;
(23) the method of nucleic acid infusion according to the above-described (22), wherein the oligosaccharide is disaccharide;
(24) the method of nucleic acid infusion according to the above-described (23), wherein the disaccharide is sucrose, maltose or lactose;
(25) the method of nucleic acid infusion according to the above-described (22), wherein the sugar alcohol is monosaccharide alcohol or oligosaccharide alcohol;
(26) the method of nucleic acid infusion according to the above-described (25), wherein the oligosaccharide alcohol is disaccharide alcohol;
(27) the method of nucleic acid infusion according to the above-described (25), wherein the monosaccharide alcohol is mannitol, sorbitol, xylitol or erythritol;
(28) the method of nucleic acid infusion according to the above-described (26), wherein the disaccharide alcohol is maltitol, lactitol or reduced palatinose;
(29) the method of nucleic acid infusion according to the above-described (22), wherein the diol is a glycol derivative;
(30) the method of nucleic acid infusion according to the above-described (22), wherein the triol is a glycerol derivative;
(31) the method of nucleic acid infusion according to the above-described (22), wherein the polyol is a polyglycol derivative;
(32) the method of nucleic acid infusion according to the above-described (29), wherein the glycol derivative is ethylene glycol;
(33) the method of nucleic acid infusion according to the above-described (30), wherein the glycerol derivative is glycerol;
(34) the method of nucleic acid infusion according to the above-described (31), wherein the polyglycol derivative is polyethylene glycol;
(35) the method of nucleic acid infusion according to the above-described (34), wherein the polyethylene glycol has a molecular weight of 2000 or less;
(36) the method of nucleic acid infusion according to the above-described (35), wherein the polyethylene glycol is PEG200, PEG300, PEG400, PEG600, PEG1000, PEG1500, PEG1540 or PEG2000;
(37) the method of nucleic acid infusion according to the above-described (22), wherein the hypertonic solution comprises (a) at least one substance selected from sucrose, mannitol, sorbitol and xylitol and (b) at least one substance selected from PEG200, PEG300, PEG400, PEG600, PEG1000, PEG1500, PEG1540, PEG2000 and glycerol;
(38) the method of nucleic acid infusion according to the above-described (37), wherein the hypertonic solution comprises (a) sucrose, mannitol, sorbitol or xylitol and (b) PEG200, PEG300, PEG400, PEG600, PEG1000, PEG1500, PEG1540, PEG2000 or glycerol;
(39) the method of nucleic acid infusion according to the above-described (6), wherein the hypertonic solution comprises one substance which is oligosaccharide or sugar alcohol;
(40) the method of nucleic acid infusion according to the above-described (39), wherein the oligosaccharide is disaccharide;
(41) the method of nucleic acid infusion according to the above-described (40), wherein the disaccharide is sucrose, maltose or lactose;
(42) the method of nucleic acid infusion according to the above-described (39), wherein the sugar alcohol is monosaccharide alcohol or oligosaccharide alcohol;
(43) the method of nucleic acid infusion according to the above-described (42), wherein the oligosaccharide alcohol is disaccharide alcohol;
(44) the method of nucleic acid infusion according to the above-described (42), wherein the monosaccharide alcohol is mannitol, sorbitol, xylitol or erythritol;
(45) the method of nucleic acid infusion according to the above-described (43), wherein the disaccharide alcohol is maltitol, lactitol or reduced palatinose;
(46) the method of nucleic acid infusion according to the above-described (39), wherein the hypertonic solution comprises sucrose, mannitol, sorbitol, xylitol or maltitol;
(47) the method of nucleic acid infusion according to any of the above-described (1) to (46), wherein the oligosaccharide and/or the polyhydric alcohol present in the hypertonic solution has a total molarity of 0.29 M to 3 M;
(48) the method of nucleic acid infusion according to the above-described (47), wherein the oligosaccharide and/or the polyhydric alcohol present in the hypertonic solution has a total molarity of 0.5 M to 2.1 M;
(49) the method of nucleic acid infusion according to any of the above-described (2) to (48), wherein the hypotonic solution has isotonic osmotic pressure or lower;
(50) a reagent for nucleic acid infusion, comprising as an ingredient at least one substance which is oligosaccharide or polyhydric alcohol;
(51) the reagent for nucleic acid infusion according to the above-described (50), wherein the reagent is used in a method of nucleic acid infusion according to any of the above-described (1) to (49);
(52) the reagent for nucleic acid infusion according to the above-described (50) or (51), wherein the oligosaccharide is disaccharide;
(53) the reagent for nucleic acid infusion according to the above-described (52), wherein the disaccharide is sucrose, maltose or lactose;
(54) the reagent for nucleic acid infusion according to the above-described (50) or (51), wherein the polyhydric alcohol is diol, triol, polyol or sugar alcohol;
(55) the reagent for nucleic acid infusion according to the above-described (54), wherein the diol is a glycol derivative;
(56) the reagent for nucleic acid infusion according to the above-described (54), wherein the triol is a glycerol derivative;
(57) the reagent for nucleic acid infusion according to the above-described (54), wherein the polyol is a polyglycol derivative;
(58) the reagent for nucleic acid infusion according to the above-described (54), wherein the sugar alcohol is monosaccharide alcohol or oligosaccharide alcohol;
(59) the reagent for nucleic acid infusion according to the above-described (58), wherein the oligosaccharide alcohol is disaccharide alcohol;
(60) the reagent for nucleic acid infusion according to the above-described (55), wherein the glycol derivative is ethylene glycol;
(61) the reagent for nucleic acid infusion according to the above-described (56), wherein the glycerol derivative is glycerol;
(62) the reagent for nucleic acid infusion according to the above-described (57), wherein the polyglycol derivative is polyethylene glycol;
(63) the reagent for nucleic acid infusion according to the above-described (62), wherein the polyethylene glycol has a molecular weight of 2000 or less;
(64) the reagent for nucleic acid infusion according to the above-described (63), wherein the polyethylene glycol is PEG200, PEG300, PEG400, PEG600, PEG1000, PEG1500, PEG1540 or PEG2000;
(65) the reagent for nucleic acid infusion according to the above-described (58), wherein the monosaccharide alcohol is mannitol, sorbitol, xylitol or erythritol;
(66) the reagent for nucleic acid infusion according to the above-described (59), wherein the disaccharide alcohol is maltitol, lactitol or reduced palatinose;
(67) the reagent for nucleic acid infusion according to the above-described (50) or (51), wherein the reagent comprises as ingredients (a) at least one substance which is oligosaccharide or sugar alcohol and (b) at least one substance which is diol, triol or polyol;
(68) the reagent for nucleic acid infusion according to the above-described (67), wherein the oligosaccharide is disaccharide;
(69) the reagent for nucleic acid infusion according to the above-described (68), wherein the disaccharide is sucrose, maltose or lactose;
(70) the reagent for nucleic acid infusion according to the above-described (67), wherein the sugar alcohol is monosaccharide alcohol or oligosaccharide alcohol;
(71) the reagent for nucleic acid infusion according to the above-described (70), wherein the oligosaccharide alcohol is disaccharide alcohol;
(72) the reagent for nucleic acid infusion according to the above-described (70), wherein the monosaccharide alcohol is mannitol, sorbitol, xylitol or erythritol;
(73) the reagent for nucleic acid infusion according to the above-described (71), wherein the disaccharide alcohol is maltitol, lactitol or reduced palatinose;
(74) the reagent for nucleic acid infusion according to the above-described (67), wherein the diol is a glycol derivative;
(75) the reagent for nucleic acid infusion according to the above-described (67), wherein the triol is a glycerol derivative;
(76) the reagent for nucleic acid infusion according to the above-described (67), wherein the polyol is a polyglycol derivative;
(77) the reagent for nucleic acid infusion according to the above-described (74), wherein the glycol derivative is ethylene glycol;
(78) the reagent for nucleic acid infusion according to the above-described (75), wherein the glycerol derivative is glycerol;
(79) the reagent for nucleic acid infusion according to the above-described (76), wherein the polyglycol derivative is polyethylene glycol;
(80) the reagent for nucleic acid infusion according to the above-described (79), wherein the polyethylene glycol has a molecular weight of 2000 or less;
(81) the reagent for nucleic acid infusion according to the above-described (80), wherein the polyethylene glycol is PEG200, PEG300, PEG400, PEG600, PEG1000, PEG1500, PEG1540 or PEG2000;
(82) the reagent for nucleic acid infusion according to the above-described (67), wherein the reagent comprises as ingredients (a) at least one substance selected from sucrose, mannitol, sorbitol and xylitol and (b) at least one substance selected from PEG200, PEG300, PEG400, PEG600, PEG1000, PEG1500, PEG1540, PEG2000 and glycerol;
(83) the reagent for nucleic acid infusion according to the above-described (82), wherein the reagent comprises as ingredients (a) sucrose, mannitol, sorbitol or xylitol and (b) PEG200, PEG300, PEG400, PEG600, PEG1000, PEG1500, PEG1540, PEG2000 or glycerol;
(84) the reagent for nucleic acid infusion according to the above-described (50) or (51), wherein the reagent comprises as an ingredient at least one substance which is oligosaccharide or sugar alcohol;
(85) the reagent for nucleic acid infusion according to the above-described (84), wherein the oligosaccharide is disaccharide;
(86) the reagent for nucleic acid infusion according to the above-described (85), wherein the disaccharide is sucrose, maltose or lactose;
(87) the reagent for nucleic acid infusion according to the above-described (84), wherein the sugar alcohol is monosaccharide alcohol or oligosaccharide alcohol;
(88) the reagent for nucleic acid infusion according to the above-described (87), wherein the oligosaccharide alcohol is disaccharide alcohol;
(89) the reagent for nucleic acid infusion according to the above-described (87), wherein the monosaccharide alcohol is mannitol, sorbitol, xylitol or erythritol;
(90) the reagent for nucleic acid infusion according to the above-described (88), wherein the disaccharide alcohol is maltitol, lactitol or reduced palatinose;
(91) the reagent for nucleic acid infusion according to the above-described (84), wherein the reagent comprises as an ingredient sucrose, mannitol, sorbitol, xylitol or maltitol;
(92) the reagent for nucleic acid infusion according to any of the above-described (50) to (91), wherein the ingredient is in the form of a solid or liquid;
(93) the reagent for nucleic acid infusion according to the above-described (92), wherein when the reagent comprises two or more ingredients which are each oligosaccharide or polyhydric alcohol, and the ingredients are each independently in the form of a solid or liquid;
(94) the reagent for nucleic acid infusion according to any of the above-described (50) to (93), wherein the reagent is prepared in advance so that the oligosaccharide and/or the polyhydric alcohol in a hypertonic solution has a total molarity of 0.29 M to 3 M;
(95) the reagent for nucleic acid infusion according to the above-described (94), wherein the reagent is prepared in advance so that the oligosaccharide and/or the polyhydric alcohol in the hypertonic solution has a total molarity of 0.5 M to 2.1 M;
(96) a kit for nucleic acid infusion, comprising a reagent according to any of the above-described (50) to (95);
(97) use of a reagent or a kit according to any of the above-described (50) to (96) in nucleic acid infusion;
(98) a nucleic acid infusion agent, comprising as an ingredient a reagent or a kit according to any of the above-described (50) to (96);
(99) a cell with a nucleic acid infused thereinto by a method of infusion according to any of the above-described (1) to (49);
(100) a macrophage-like cell line RAW264.7 cell, a hybridoma DO11.10 cell, a primary cultured hepatocyte, a primary cultured myoblast, a primary cultured muscle cell, a primary cultured adipocyte precursor cell, a primary cultured adipocyte or a primary cultured neuron with a nucleic acid infused thereinto by a method of infusion according to any of the above-described (1) to (49);
(101) Cell-derived RNA or protein prepared from a cell according to the above-described (99) or (100); and
(102) a method of functional analysis of a gene or protein associated with an infused nucleic acid, characterized by using a cell according to the above-described (99) or (100) or RNA or protein according to the above-described (101).

### BRIEF DESCRIPTION OF THE DRAWINGS

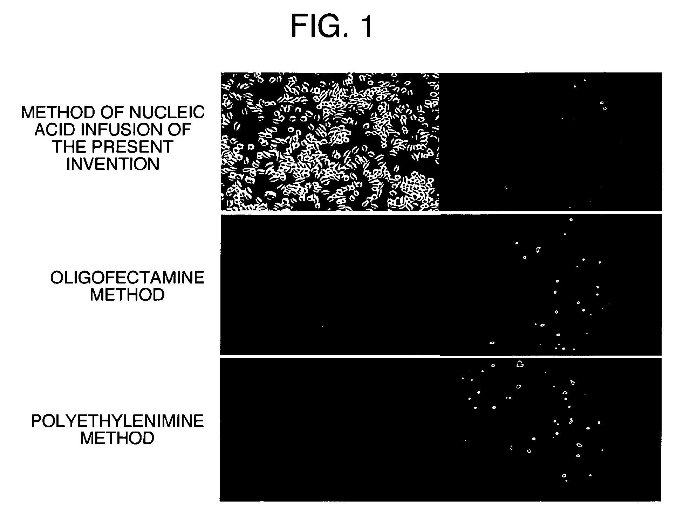

FIG. 1 shows a result of oligonucleotide infusion into murine macrophage-like cell line RAW264.7 cells by a method of nucleic acid infusion of the present invention (upper column), an Oligofectamine method (middle column) and a polyethylenimine method (lower column). In the drawing, the left and right columns show the bright-field and fluorescence images, respectively, of the infused cells;

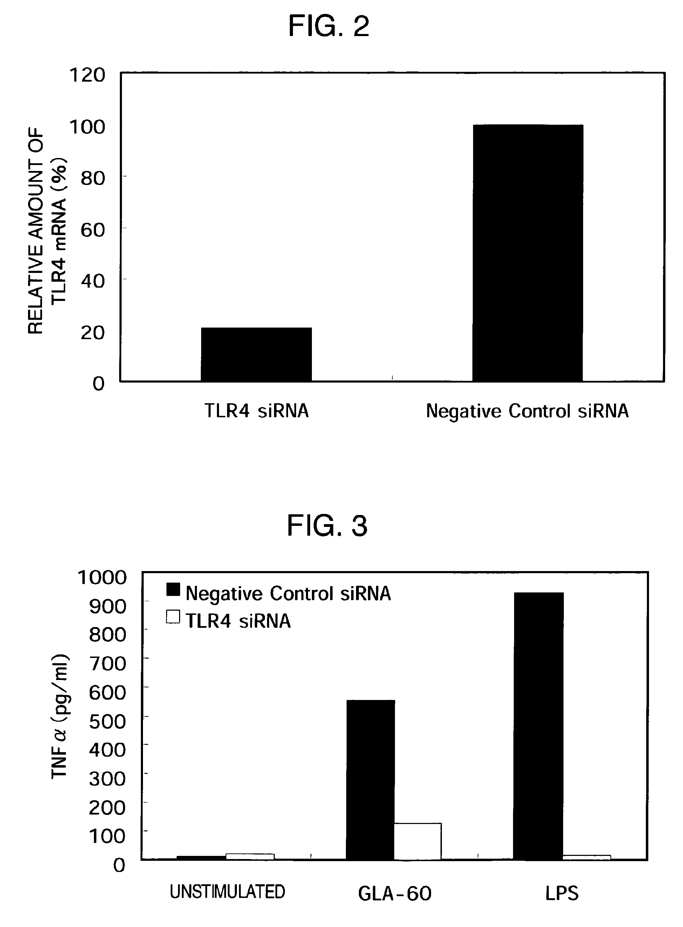

FIG. 2 shows a result of measuring siRNA effect by quantitative PCR after TLR4 siRNA infusion into RAW264.7 cells by the method of nucleic acid infusion of the present invention. In the drawing, TLR4 siRNA indicates a result of TLR4 siRNA infusion, and Negative Control siRNA indicates a result of infusion of siRNA that serves as a negative control. The vertical axis indicates the relative amount of TLR4 mRNA after normalization with the amount of GAPDH mRNA;
FIG. 3 shows a result of measuring the inhibitory effect of stimulation with LPS or GLA-60 on TNFα production after TLR4 siRNA infusion into RAW264.7 cells by the method of nucleic acid infusion of the present invention. In the drawing, TLR4 siRNA indicates a result of TLR4 siRNA infusion, and Negative Control siRNA indicates a result of infusion of siRNA that serves as a negative control. The vertical axis indicates the amount of TNFα produced;

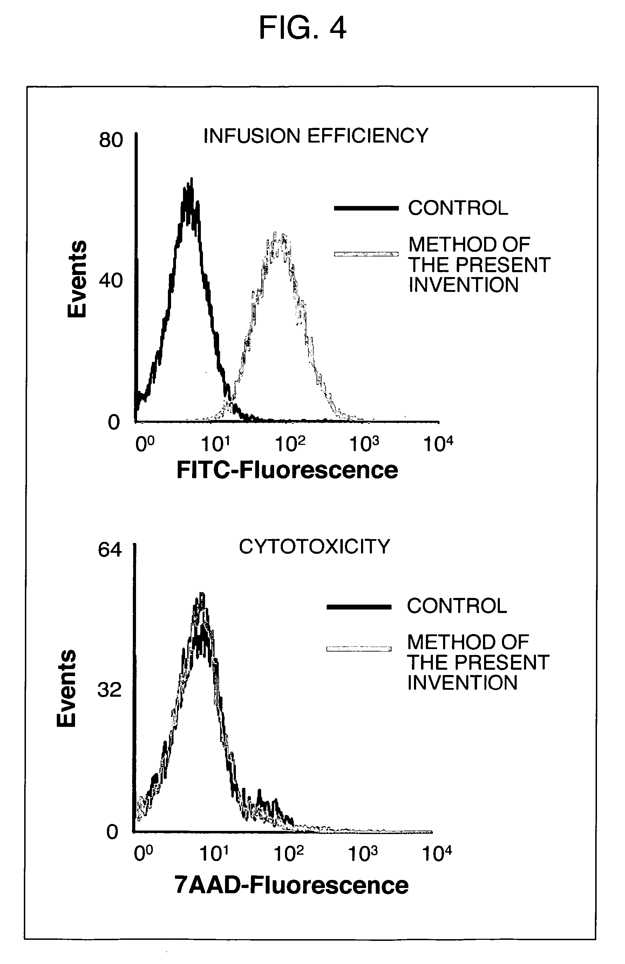

FIG. 4 shows a result of measuring infusion efficiency (upper diagram) and cytotoxicity (lower diagram) after FITC-labeled oligonucleotide infusion into RAW264.7 cells by the method of nucleic acid infusion of the present invention. The horizontal axis indicates the amount of fluorescence emitted from FITC as a marker for infusion efficiency (upper diagram) and the amount of fluorescence emitted from 7AAD as a marker for cytotoxicity (lower diagram), and the vertical axis indicates the number of events;

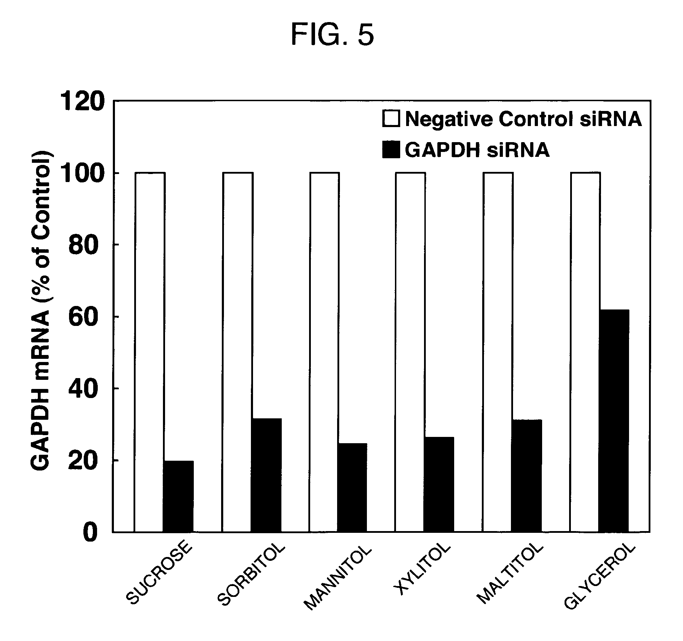

FIG. 5 shows a result of using RAW264.7 cells to measure the effect of types of oligosaccharide and polyhydric alcohol contained in a hypertonic solution on siRNA infusion into cells. In the drawing, GAPDH siRNA indicates a result of GAPDH siRNA infusion, and Negative Control siRNA indicates a result of infusion of siRNA that serves as a negative control. The vertical axis indicates the relative amount of GAPDH mRNA after normalization with the amount of cyclophilin mRNA;

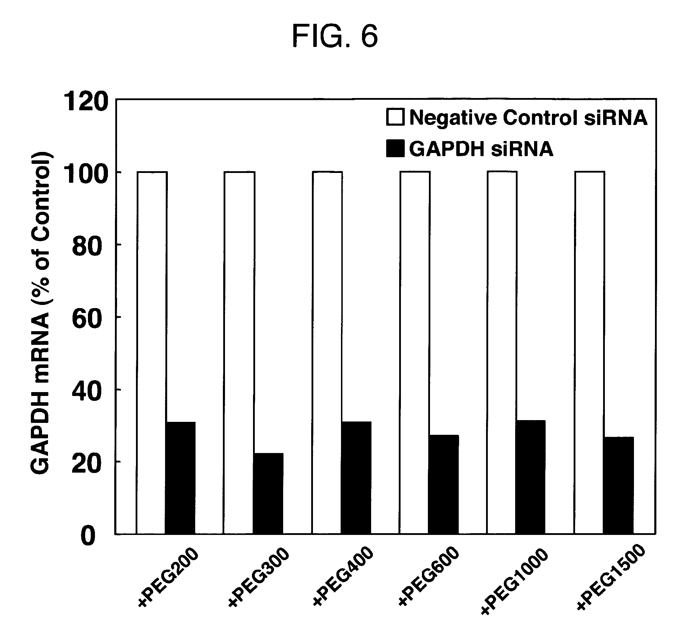

FIG. 6 shows a result of using RAW264.7 cells to measure the effect of a combination of sucrose and polyhydric alcohol (PEG) contained in a hypertonic solution on siRNA infusion into cells. In the drawing, GAPDH siRNA indicates a result of GAPDH siRNA infusion, and Negative Control siRNA indicates a result of infusion of siRNA that serves as a negative control. The vertical axis indicates the relative amount of GAPDH mRNA after normalization with the amount of cyclophilin mRNA;

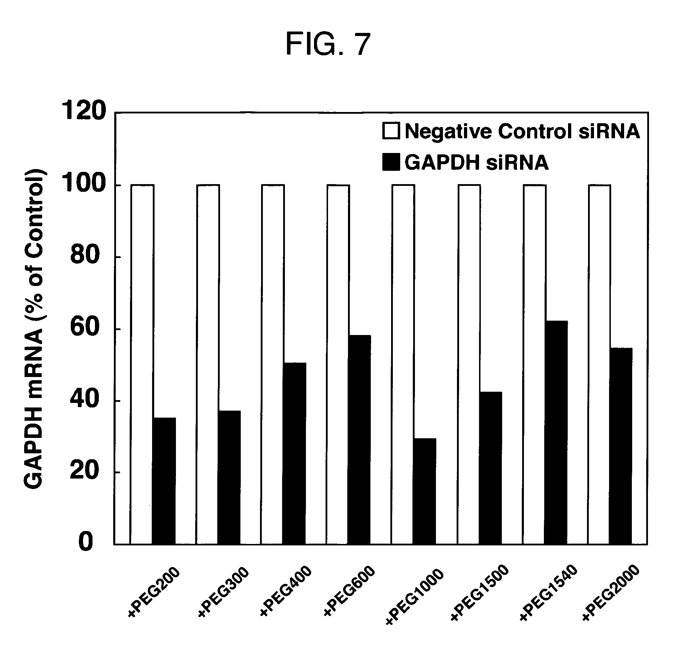

FIG. 7 shows a result of using RAW264.7 cells to measure the effect of a combination of sorbitol and polyhydric alcohol (PEG) contained in a hypertonic solution on siRNA infusion into cells. In the drawing, GAPDH siRNA indicates a result of GAPDH siRNA infusion, and Negative Control siRNA indicates a result of infusion of siRNA that serves as a negative control. The vertical axis indicates the relative amount of GAPDH mRNA after normalization with the amount of cyclophilin mRNA;

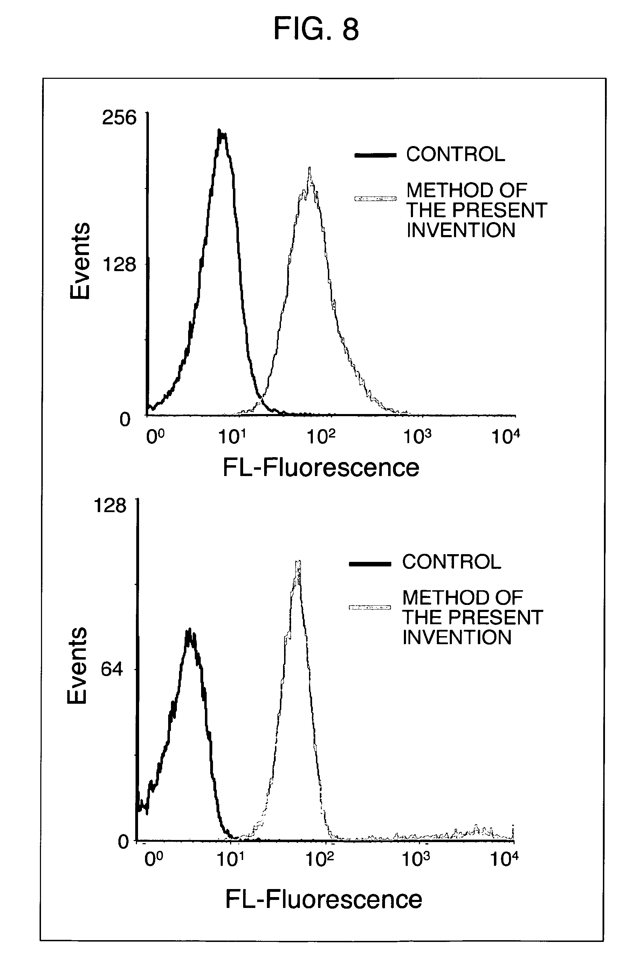

FIG. 8 shows a result of using DO11.10 cells to investigate whether the method of nucleic acid infusion of the present invention can also be applied to a nucleic acid with no electric charge. The upper diagram indicates a result of infusion of morpholino antisense oligonucleotide that is a nucleic acid analog with no electric charge, and the lower diagram indicates a result of infusion of phosphorothioate oligodeoxynucleotide that is a nucleic acid analog with an electric charge. In the upper diagram, the horizontal axis indicates the amount of fluorescence emitted from fluorescein as a marker for infusion, and the vertical axis indicates the number of events. In the lower diagram, the horizontal axis indicates the amount of fluorescence emitted from FITC as a maker for infusion, and the vertical axis indicates the number of events;

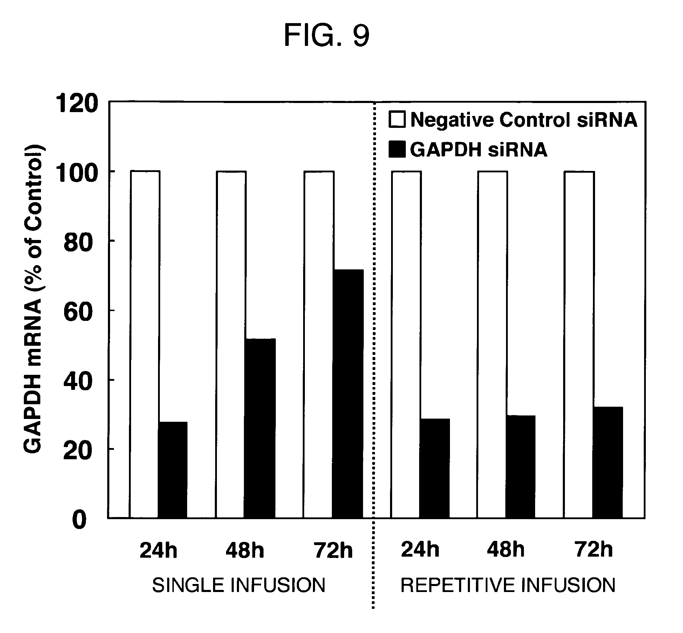

FIG. 9 shows a result of using D011.10 cells to examine the effect of siRNA repetitive infusion by the method of nucleic acid infusion of the present invention. In the drawing, GAPDH siRNA indicates a result of GAPDH siRNA infusion, and Negative Control siRNA indicates a result of infusion of siRNA that serves as a negative control. The vertical axis indicates the relative amount of GAPDH mRNA after normalization with the amount of cyclophilin mRNA;

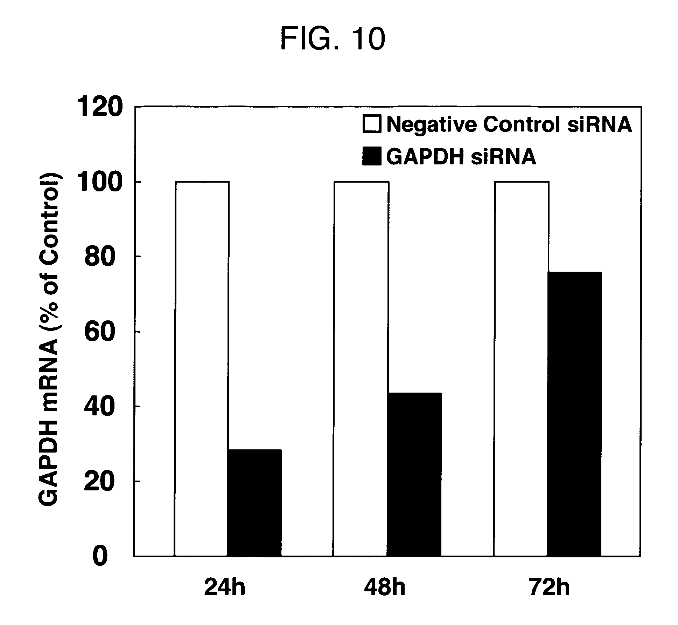

FIG. 10 shows a result of measuring infusion efficiency (knockdown activity) after siRNA infusion into primary cultured differentiated rat adipocytes by the method of nucleic acid infusion of the present invention. In the drawing, GAPDH siRNA indicates a result of GAPDH siRNA infusion, and Negative Control siRNA indicates a result of infusion of siRNA that serves as a negative control. The vertical axis indicates the relative amount of GAPDH mRNA after normalization with the amount of cyclophilin mRNA; and

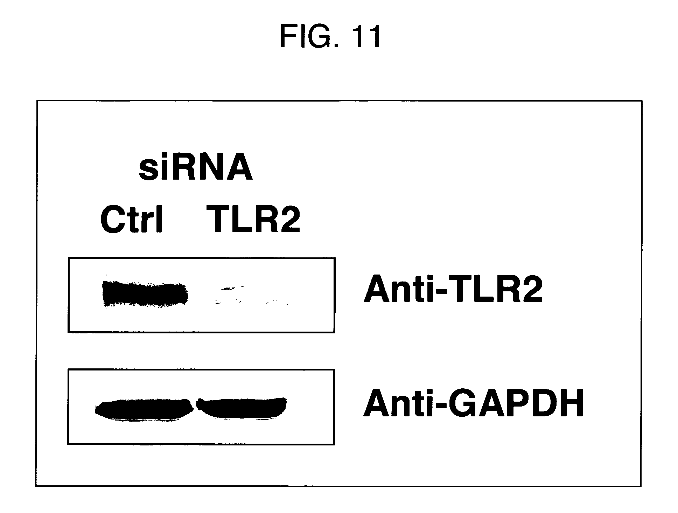

FIG. 11 shows a result of measuring infusion efficiency (knockdown activity) at a protein level after siRNA repetitive infusion into RAW264.7 cells by the method of nucleic acid infusion of the present invention. In the drawing, Ctrl indicates negative control siRNA, and TLR2 indicates siRNA for TLR2.
Anti-TLR2 indicates a TLR2 protein detected with an anti-TLR2 antibody, and Anti-GAPDH indicates a GAPDH protein detected with an anti-GAPDH antibody.

### BEST MODE FOR CARRYING OUT THE INVENTION

The present invention provides a method of nucleic acid infusion, comprising at least the following steps (a) and (b):
(a) bringing a nucleic acid, a hypertonic solution and cells into contact with each other; and
(b) lowering osmotic pressure of the hypertonic solution after the above-described step (a).

The nucleic acid used in the step (a) is not limited by types in terms of the principle of the method, and any nucleic acid can be used as the nucleic acid to be infused. That is, the nucleic acid may be any of oligonucleotide and polynucleotide and may assume any morphology of single and double strands and an analog thereof. When the nucleic acid of the present invention is single-stranded, any of sense and antisense strands can be used. When the nucleic acid of the present invention is polynucleotide, the nucleic acid may assume the morphology of a plasmid.

Preferred examples of the nucleic acid of the present invention can include oligonucleotide. Oligonucleotide to be infused is not limited by types in terms of the principle of the method, and any of single-stranded oligonucleotide, double-stranded oligonucleotide and an analog thereof can be used. Specifically, the oligonucleotide can be exemplified by deoxyribonucleotide (DNA), ribonucleotide (RNA), phosphorothioate oligodeoxynucleotide (see Example 1), a 2'-O-(2-methoxy)ethyl-modified nucleic acid (2'-MOE-modified nucleic acid), small interfering RNA (siRNA; see Examples 2 and 3), a locked nucleic acid (LNA; Singh et al., Chem. Commun., 455, 1998), a peptide nucleic acid (PNA; Nielsen et al., Science, 254, 1497, 1991), or morpholino antisense oligonucleotide (Summerton and Weller, Antisense & Nucleic Acid Drug Development, 7, 187, 1997).

The concentration of the nucleic acid of the present invention in the hypertonic solution may be any concentration at which the nucleic acid can be infused into cells. The concentration can include usually a range of 0.01 µM to 1 mM, preferably 1 µM to 100 µM.

The hypertonic solution of the present invention that can be used in the step (a) may be any solution that maintains isotonic osmotic pressure or higher osmotic pressure and allows the cellular uptake of the nucleic acid by bringing the hypertonic solution containing the nucleic acid and cells into contact with each other. Preferred examples of the hypertonic solution include a hypertonic solution comprising at least one substance which is oligosaccharide or polyhydric alcohol.

In this context, concrete examples of the oligosaccharide include disaccharide, trisaccharide, tetrasaccharide, pentasaccharide or decasaccharide, with disaccharide preferred. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

Concrete examples of the above-described polyhydric alcohol include diol, triol, polyol or sugar alcohol, with triol, polyol or sugar alcohol more preferred.

In this context, the diol includes a glycol derivative. The glycol derivative is exemplified by ethylene glycol.

The triol includes a glycerol derivative. The glycerol derivative includes glycerol.

The polyol includes a polyglycol derivative. The polyglycol derivative includes polyethylene glycol.

The polyethylene glycol may have any molecular weight not greater than 2000. Specifically, the polyethylene glycol is exemplified by PEG200, PEG300, PEG400, PEG600, PEG1000, PEG1500, PEG1540 and PEG2000.

The above-described sugar alcohol includes monosaccharide alcohol or oligosaccharide alcohol.

The monosaccharide alcohol includes mannitol, sorbitol, xylitol or erythritol.

The above-described oligosaccharide alcohol includes disaccharide alcohol. The disaccharide alcohol includes maltitol, lactitol or reduced palatinose.

The hypertonic solution used in the method of nucleic acid infusion of the present invention may comprise at least one substance which is a variety of oligosaccharides or polyhydric alcohols described above. Specifically, the hypertonic solution may comprise at least one substance arbitrarily selected from among the substances listed above, for example, sucrose, maltose, lactose, trehalose, levanbiose, chitobiose, vicianose, sambubiose, melibiose, epicellobiose, turanose, rutinose, robinobiose, ethylene glycol, glycerol, polyethylene glycol (PEG having a molecular weight of 2000 or less, e.g., PEG200, PEG300, PEG400, PEG600, PEG1000, PEG1500, PEG1540 and PEG2000), mannitol, sorbitol, xylitol, erythritol, maltitol, lactitol and reduced palatinose. To be more specific, the hypertonic solution is exemplified by a hypertonic solution comprising one substance, two substances, three substances or four or more substances arbitrarily selected from among the substances listed above. In this case, it is necessary to prepare the hypertonic solution so that the oligosaccharide and/or the polyhydric alcohol present in the hypertonic solution has a total molarity of 0.29 M to 3 M for allowing the cellular uptake of the nucleic acid when the hypertonic solution, the nucleic acid and cells are brought into contact with each other. The total molarity may be any molarity that falls within this range and includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

The hypertonic solution used in the method of nucleic acid infusion of the present invention may comprise at least one substance which is oligosaccharide or polyhydric alcohol, as described above. Alternatively, the hypertonic solution may comprise only one substance or two or more substances which are each oligosaccharide or polyhydric alcohol. When the hypertonic solution comprises two or more substances which are each oligosaccharide or polyhydric alcohol, it is preferred that the hypertonic solution should comprise (1) at least one substance which is oligosaccharide or sugar alcohol and (2) at least one substance which is diol, triol or polyol.

In this context, concrete examples of the oligosaccharide used include disaccharide, trisaccharide, tetrasaccharide, pentasaccharide or decasaccharide, with disaccharide preferred. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

The above-described sugar alcohol includes monosaccharide alcohol or oligosaccharide alcohol.

The monosaccharide alcohol includes mannitol, sorbitol, xylitol or erythritol.

The above-described oligosaccharide alcohol includes disaccharide alcohol.

The disaccharide alcohol includes maltitol, lactitol or reduced palatinose.

The above-described diol includes a glycol derivative. The glycol derivative includes ethylene glycol.

The triol includes a glycerol derivative. The glycerol derivative includes glycerol.

The polyol includes a polyglycol derivative. The polyglycol derivative includes polyethylene glycol.

The polyethylene glycol may have any molecular weight not greater than 2000. Specifically, the polyethylene glycol is exemplified by PEG200, PEG300, PEG400, PEG600, PEG1000, PEG1500, PEG1540 and PEG2000.

A preferred combination of (1) at least one substance which is oligosaccharide or sugar alcohol and (2) at least one substance which is diol, triol or polyol contained in the hypertonic solution of the present invention as described above is exemplified by a combination between (1) at least one substance selected from sucrose, mannitol, sorbitol and xylitol and (2) at least one substance selected from PEG200, PEG300, PEG400, PEG600, PEG1000, PEG1500, PEG1540, PEG2000 and glycerol.

More preferably, the hypertonic solution comprising the combination is exemplified by a hypertonic solution comprising (1) one substance which is oligosaccharide or sugar alcohol and (2) one substance which is diol, triol or polyol and specifically exemplified by a hypertonic solution comprising (1) sucrose, mannitol, sorbitol or xylitol and (2) PEG200, PEG300, PEG400, PEG600, PEG1000, PEG1500, PEG1540, PEG2000 or glycerol.

Such a hypertonic solution of the present invention comprising two or more substances which are each oligosaccharide or polyhydric alcohol may have each ingredient at any molarity, as long as the hypertonic solution is prepared so that the oligosaccharide and/or the polyhydric alcohol present in the hypertonic solution has a total molarity of 0.29 M to 3 M. The total molarity includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

When the hypertonic solution of the present invention comprises two substances which are each oligosaccharide or polyhydric alcohol, the molarity of each ingredient may be any value as described above, as long as total molarity of the two substances falls within the above-described range. In this case, the molarity of (1) one substance which is oligosaccharide or sugar alcohol can be exemplified by a range of 0.5 M to 2 M, and the molarity of (2) one substance which is diol, triol or polyol can be exemplified by 0.1 M.

When the hypertonic solution used in the method of nucleic acid infusion of the present invention comprises only one substance which is oligosaccharide or polyhydric alcohol, it is preferred to select the one substance from oligosaccharide or sugar alcohol.

In this context, concrete examples of the oligosaccharide used include disaccharide, trisaccharide, tetrasaccharide, pentasaccharide or decasaccharide, with disaccharide preferred. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

The above-described sugar alcohol includes monosaccharide alcohol or oligosaccharide alcohol.

The monosaccharide alcohol includes mannitol, sorbitol, xylitol or erythritol.

The above-described oligosaccharide alcohol includes disaccharide alcohol.

The disaccharide alcohol includes maltitol, lactitol or reduced palatinose.

The hypertonic solution of the present invention comprising only one substance which is oligosaccharide or sugar alcohol as described above is preferably a hypertonic solution comprising sucrose, mannitol, sorbitol, xylitol or maltitol.

Such a hypertonic solution of the present invention comprising only one substance which is oligosaccharide or polyhydric alcohol may be prepared so that the oligosaccharide or the polyhydric alcohol present in the hypertonic solution has a molarity of 0.29 M to 3 M. The molarity includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

Any solution that comprises at least one substance which is oligosaccharide or polyhydric alcohol and allows the cellular uptake of the nucleic acid by bringing the hypertonic solution, the nucleic acid and cells into contact with each other can be used as the hypertonic solution of the present invention. Specifically, it is preferred that the hypertonic solution of the present invention should comprise a culture solution, a buffer solution and/or serum. It is more preferred that the hypertonic solution of the present invention should comprise all of a culture solution, a buffer solution and serum.

In this context, the culture solution used may be any culture solution suitable for each cell. The culture solution is exemplified by RPMI1640 (Invitrogen), DULBECCO'S MODIFIED EAGLE MEDIA (Invitrogen), F-10 Nutrient Mixture (Invitrogen), F-12 Nutrient Mixture (Invitrogen), Iscove's Modified Dulbecco's Media (Invitrogen) or MINIMUM ESSENTIAL MEDIA (Invitrogen).

The buffer solution is exemplified by a HEPES buffer solution (Sigma), a TRIS buffer solution (Sigma), or a PBS buffer solution (Invitrogen). The concentration of the buffer solution in the hypertonic solution may be any molarity. The molarity includes a range of preferably 0 M to 1 M, more preferably 1 mM to 100 mM, most preferably 1 mM to 10 mM.

The serum is exemplified by fetal bovine serum, bovine serum, calf serum or equine serum. The concentration of the serum in the hypertonic solution may be any concentration. The concentration includes a range of preferably 0 to 20% (v/v), more preferably 5 to 10% (v/v).

Total molarity of the whole hypertonic solution of the present invention may be within a range of isotonic to 3 M.

The cell used in the method of nucleic acid infusion of the present invention is not limited by applied cell types in terms of the principle of the method. That is, as shown in Examples, the method of nucleic acid infusion of the present invention has allowed nucleic acid infusion into a variety of cell types that are hardly infused with a nucleic acid heretofore. Thus, the method of nucleic acid infusion of the present invention can be applied to any cell type without limitations.

Specifically, the method of nucleic acid infusion can be applied to not only a cell to which usual nucleic acid infusion is readily applied, but also a hybridoma, a primary cultured hepatocyte, a primary cultured myoblast, a primary cultured muscle cell, a primary cultured brown adipocyte precurson cell, a primary cultured brown adipocyte, a primary cultured white adipocyte precursor cell, a primary cultured white adipocyte or a primary cultured neuron or the like.

Furthermore, the method of the present invention is not limited by the origins of cells in terms of the principle of the method and can be applied to not only animal cells, but also plant cells, insect cells or nematode cells including protoplast forms thereof.

The method of nucleic acid infusion of the present invention is characterized by comprising (a) bringing a nucleic acid, a hypertonic solution and cells into contact with each other and (b) lowering osmotic pressure of the above-described hypertonic solution after the step (a).

In this context, specifically, an isotonic solution or a solution of lower osmotic pressure (hypotonic solution) and the cells may be brought into contact with each other for lowering the osmotic pressure of the hypertonic solution at the above-described step (b). It is more preferred to lower the osmotic pressure by bringing the hypotonic solution and the cells into contact with each other. The specific composition of the hypotonic solution can include a hypotonic solution of a medium and water (preferably DEPC-treated water) mixed at a ratio of 6:4, or water (e.g., distilled water and DEPC-treated water).

Next, a specific example of the method of nucleic acid infusion of the present invention will be illustrated.

At first, cells to be infused are cultured. The cells may be cultured with a culture solution suitable for each cell under usual conditions (37°C, 5% CO₂). The number (density) of cells may be the number of cells at which each cell can favorably be grown.

A hypertonic solution and a hypotonic solution may appropriately be prepared to have the above-described composition of the hypertonic solution and the hypotonic solution of the present invention.

Nucleic acid infusion into the cells is performed as illustrated below. At first, a culture supernatant was removed from the cells, which are then brought into contact with a nucleic acid and the hypertonic solution. The contact may be performed by any method by which the nucleic acid and the cells are eventually brought into contact with each other in the hypertonic solution. Specifically, the method is exemplified by a method in which the hypertonic solution containing the nucleic acid is prepared in advance and then added to the above-described cells and a method in which the nucleic acid (a solution containing the nucleic acid) is supplemented initially with the cells and subsequently with the hypertonic solution.

The cells are then incubated for an appropriate period of time. The incubation time is preferably approximately 2 minutes to 20 minutes, more preferably approximately 10 minutes to 15 minutes. After the hypertonic solution containing the nucleic acid is removed, the hypotonic solution is added. Alternatively, water is added without the removal of the hypertonic solution, to render the solution isotonic. The resulting solution may be incubated for an appropriate period of time, or otherwise after the removal of the added hypotonic solution, the hypotonic solution may be added again, followed by incubation for an appropriate period of time. The incubation time is preferably approximately 2 minutes to 10 minutes. An incubation temperature after the addition of the above-described hypertonic solution and hypotonic solution is not particularly limited, as long as the resulting solution is kept in a liquid state at the temperature. The incubation temperature includes a range of preferably 0°C to 42°C, more preferably room temperature to 37°C.

The hypotonic solution is then removed, and a usual growth medium is added. The nucleic acid infusion is accomplished by these procedures. These procedures for infusion can be repeated.

The method of nucleic acid infusion of the present invention allows oligonucleotide infusion with higher efficiency than ever (50% or more, preferably 60% or more, more preferably 70% or more, even more preferably 80% or more efficiency). In addition, the method of the present invention allows efficient oligonucleotide infusion into cell types that are hardly infused with a nucleic acid by conventional techniques. Since the method of the present invention does not employ adjuvants (such as liposomes and polyethylenimine) conventionally used, the method overcomes the problems of conventional methods such as cytotoxicity and immunoadjuvant activity. Therefore, the present invention makes it possible to efficiently infuse oligonucleotide into not only adherent cell lines but also primary cultured cells and lymphocytes that are highly sensitive to cytotoxicity.

The present invention provides a reagent for nucleic acid infusion used in the above-described method of nucleic acid infusion of the present invention.

The reagent for nucleic acid infusion of the present invention is characterized by comprising as an ingredient at least one substance which is oligosaccharide or polyhydric alcohol.

The oligosaccharide and the polyhydric alcohol can be prepared by a usual approach conventionally known in the art.

In this context, concrete examples of the oligosaccharide include disaccharide. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

The above-described polyhydric alcohol includes diol, triol, polyol or sugar alcohol, with triol, polyol or sugar alcohol more preferred.

In this context, the diol includes a glycol derivative. The glycol derivative is exemplified by ethylene glycol.

The triol includes a glycerol derivative. The glycerol derivative includes glycerol.

The polyol includes a polyglycol derivative. The polyglycol derivative includes polyethylene glycol.

The polyethylene glycol may have any molecular weight not greater than 2000. Specifically, the polyethylene glycol is exemplified by PEG200, PEG300, PEG400, PEG600, PEG1000, PEG1500, PEG1540 and PEG2000.

The above-described sugar alcohol includes monosaccharide alcohol or oligosaccharide alcohol. The monosaccharide alcohol includes mannitol, sorbitol, xylitol or erythritol.

The above-described oligosaccharide alcohol includes disaccharide alcohol.

The disaccharide alcohol includes maltitol, lactitol or reduced palatinose.

The reagent for nucleic acid infusion of the present invention may comprise at least one substance which is a variety of oligosaccharides or polyhydric alcohols listed above. Specifically, the reagent for nucleic acid infusion may comprise at least one substance arbitrarily selected from among the substances listed above, for example, sucrose, maltose, lactose, trehalose, levanbiose, chitobiose, vicianose, sambubiose, melibiose, epicellobiose, turanose, rutinose, robinobiose, ethylene glycol, glycerol, polyethylene glycol (PEG having a molecular weight of 2000 or less, e.g., PEG200, PEG300, PEG400, PEG600, PEG1000, PEG1500, PEG1540 and PEG2000), mannitol, sorbitol, xylitol, erythritol, maltitol, lactitol and reduced palatinose. To be more specific, the reagent for nucleic acid infusion may comprise one substance, two substances, three substances or four or more substances arbitrarily selected from among the substances listed above. In this case, the reagent for nucleic acid infusion of the present invention may comprise each ingredient at any concentration, as long as the reagent for nucleic acid infusion of the present invention is prepared so that total molarity of the oligosaccharide and/or the polyhydric alcohol present in a hypertonic solution prepared with the reagent for nucleic acid infusion falls within a range of 0.29 M to 3 M for allowing the cellular uptake of a nucleic acid when the hypertonic solution, the nucleic acid and cells are brought into contact with each other. The total molarity of the oligosaccharide and/or the polyhydric alcohol in the hypertonic solution may be any molarity that falls within the above-described range and includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

The reagent for nucleic acid infusion of the present invention may comprise at least one substance which is oligosaccharide or polyhydric alcohol, as described above. The reagent for nucleic acid infusion may comprise only one substance or two ore more substances which are each oligosaccharide or polyhydric alcohol. When the reagent for nucleic acid infusion comprises two or more substances which are each oligosaccharide or polyhydric alcohol, it is preferred that the reagent should comprise (1) at least one substance which is oligosaccharide or sugar alcohol and (2) at least one substance which is diol, triol or polyol.

In this context, the oligosaccharide used includes disaccharide. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

The above-described sugar alcohol includes monosaccharide alcohol or oligosaccharide alcohol.

The monosaccharide alcohol includes mannitol, sorbitol, xylitol or erythritol.

The above-described oligosaccharide alcohol includes disaccharide alcohol.

The disaccharide alcohol includes maltitol, lactitol or reduced palatinose.

The above-described diol includes a glycol derivative. The glycol derivative includes ethylene glycol.

The triol includes a glycerol derivative. The glycerol derivative includes glycerol.

The polyol includes a polyglycol derivative. The polyglycol derivative includes polyethylene glycol (PEG).

The polyethylene glycol may have any molecular weight not greater than 2000. Specifically, the polyethylene glycol is exemplified by PEG200, PEG300, PEG400, PEG600, PEG1000, PEG1500, PEG1540 and PEG2000.

A preferred combination of (1) at least one substance which is oligosaccharide or sugar alcohol and (2) at least one substance which is diol, triol or polyol contained as an ingredient in the reagent for nucleic acid infusion of the present invention as described above is exemplified by a combination between (1) at least one substance selected from sucrose, mannitol, sorbitol and xylitol and (2) at least one substance selected from PEG200, PEG300, PEG400, PEG600, PEG1000, PEG1500, PEG1540, PEG2000 and glycerol.

More preferably, the reagent for nucleic acid infusion comprising the combination is exemplified by a reagent for nucleic acid infusion comprising (1) one substance which is oligosaccharide or sugar alcohol and (2) one substance which is diol, triol or polyol and specifically exemplified by a reagent for nucleic acid infusion comprising as an ingredient (1) sucrose, mannitol, sorbitol or xylitol and (2) PEG200, PEG300, PEG400, PEG600, PEG1000, PEG1500, PEG1540, PEG2000 or glycerol.

Such a reagent for nucleic acid infusion of the present invention comprising two or more substances which are each oligosaccharide or polyhydric alcohol may comprise each ingredient at any molarity, as long as the reagent for nucleic acid infusion of the present invention is prepared so that the oligosaccharide and/or the polyhydric alcohol in a hypertonic solution (i.e., the hypertonic solution of the present invention used in nucleic acid infusion) prepared with the reagent for nucleic acid infusion has a total molarity of 0.29 M to 3M for allowing the cellular uptake of a nucleic acid when the hypertonic solution, the nucleic acid, and cells are brought into contact with each other. The total molarity of the oligosaccharide and/or the polyhydric alcohol in the hypertonic solution includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

When the reagent for nucleic acid infusion of the present invention comprises two substances which are each oligosaccharide or polyhydric alcohol, the molarity of each ingredient may be any value as described above, as long as total molarity of these two ingredients in the hypertonic solution falls within the above-described range. In this case, molarity of (1) one substance which is oligosaccharide or sugar alcohol in the hypertonic solution can be exemplified by a range of 0.5 M to 2 M, and molarity of (2) one substance which is diol, triol or polyol in the hypertonic solution can be exemplified by 0.1 M.

When the reagent for nucleic acid infusion of the present invention comprises only one substance which is oligosaccharide or polyhydric alcohol, it is preferred that the reagent should comprise one substance which is oligosaccharide or sugar alcohol.

In this context, concrete examples of the oligosaccharide used include disaccharide, trisaccharide, tetrasaccharide, pentasaccharide or decasaccharide, with disaccharide preferred. The disaccharide includes sucrose, maltose, lactose, trehalose, levanbiose, chitobiose, vicianose, sambubiose, melibiose, epicellobiose, turanose, rutinose or robinobiose, with sucrose, maltose or lactose preferred.

The above-described sugar alcohol includes monosaccharide alcohol or oligosaccharide alcohol.

The monosaccharide alcohol includes mannitol, sorbitol, xylitol or erythritol.

The above-described oligosaccharide alcohol includes disaccharide alcohol.

The disaccharide alcohol includes maltitol, lactitol or reduced palatinose.

It is preferred that the reagent for nucleic acid infusion of the present invention comprising only one substance which is oligosaccharide or sugar alcohol as described above should comprise sucrose, mannitol, sorbitol, xylitol or maltitol.

Such a reagent for nucleic acid infusion of the present invention comprising only one substance which is oligosaccharide or polyhydric alcohol may comprise the substance (ingredient) at any molarity, as long as the oligosaccharide or the polyhydric alcohol present in a hypertonic solution (i.e., the hypertonic solution of the present invention used in nucleic acid infusion) prepared with the reagent for nucleic acid infusion of the present invention has a molarity of 0.29 M to 3M for allowing the cellular uptake of a nucleic acid when the hypertonic solution, the nucleic acid, and cells are brought into contact with each other. The molarity of the oligosaccharide or the polyhydric alcohol in the hypertonic solution includes a range of preferably 0.35 M to 2.5 M, more preferably 0.35 M to 2.1 M, even more preferably 0.5 M to 2.1 M.

Although suitable molarity varies depending on cells, molarity suitable for a desired cell can readily be determined, for example, by using three concentrations of 1 M, 1.5 M and 2 M to investigate infusion into the cell.

Each ingredient in the reagent for nucleic acid infusion of the present invention may be in the form of a solid or liquid. In this context, the solid form refers to waxy (e.g., wax) form, vaseline-like form, flake-like form, crystallized form, powdery form, and the like. Alternatively, the liquid form refers to highly viscous form, low viscous form, nonviscous form, and the like.

When the reagent for nucleic acid infusion comprises two or more ingredients (substances) belonging to the category of oligosaccharide or polyhydric alcohol, the ingredients may be each in independent solid or liquid form or may be formed into a solid or liquid mixture.

In this context, the independent solid form means that the ingredients are each individually solidified. The ingredients may be enclosed together in one container or enclosed in separate containers. The above-described independent liquid form means that each of the ingredients is enclosed in a liquid state in a separate container.

The above-described solid mixture means that the ingredients are mixed in a solid state, or are solidified with then mixed, and then enclosed in one container. The above-described liquid mixture means that the ingredients are mixed in a liquid state and enclosed in one container, or one of the ingredients in a liquid state and the other of the ingredients in a solid state are mixed together and enclosed in one container.

Specifically, for example, (1) at least one substance which is oligosaccharide or sugar alcohol is crystallized, and (2) at least one substance which is diol, triol or polyol is in waxy (wax) form. In this case, these two substances may be enclosed in one container. To be more specific, for example, (1) sucrose in crystallized form and (2) polyethylene glycol in waxy (wax) form are enclosed in one container.

Each of the ingredients in solid form may be dissolved by a method (e.g., heating and dilution) suitable for each of them. The ingredients may be prepared so that the hypertonic solution of the present invention finally exhibits the molarity shown above by the addition of a culture solution, a buffer solution and/or serum.

The reagent for nucleic acid infusion of the present invention as described above can be one of ingredients in a kit for nucleic acid infusion. The kit may consist of only the above-described reagent for nucleic acid infusion of the present invention or may comprise the reagent for nucleic acid infusion of the present invention as well as other ingredients. Other ingredients in the kit include fluorescently labeled oligonucleotide and positive control siRNA.

The reagent and the kit for nucleic acid infusion of the present invention can be used as a nucleic acid infusion agent for allowing nucleic acid infusion.

The present invention provides a cell infused with a nucleic acid by the above-described method of nucleic acid infusion of the present invention.

As described above, the method of nucleic acid infusion of the present invention has allowed nucleic acid infusion into cells with high efficiency. The method of the present invention has also allowed nucleic acid infusion into a variety of cells that are hardly infused with a nucleic acid heretofore. The present invention provides a cell infused with a nucleic acid by such a method of nucleic acid infusion of the present invention.

The cell of the present invention may be any cell that is infused with a nucleic acid by the method of nucleic acid infusion of the present invention. Examples of the cell include a cell obtained by infusing a nucleic acid into a macrophage-like cell line RAW264.7 cell, a hybridoma Doll.10 cell, a primary cultured hepatocyte, a primary cultured myoblast, a primary cultured muscle cell, a primary cultured adipocyte precursor cell, a primary cultured adipocyte or a primary cultured neuron by the method of nucleic acid infusion of the present invention.

Preferably, the cell includes a cell obtained by infusing a nucleic acid into a cell that is hardly infused with a nucleic acid heretofore, that is, a macrophage-like cell line RAW264.7 cell, a hybridoma D011.10 cell, a primary cultured hepatocyte, a primary cultured myoblast, a primary cultured muscle cell, a primary cultured adipocyte precursor cell, a primary cultured adipocyte or a primary cultured neuron by the method of nucleic acid infusion of the present invention.

These cells can be constructed by the above-described method of nucleic acid infusion of the present invention.

The present invention provides RNA or a protein derived from a cell prepared from the cell of the present invention. The RNA or the protein derived from the cell is prepared by a method already known in the art and can readily be prepared with reference to, for example, Molecular Cloning 3^{rd} Edition (Cold Spring Harbor Press), Current Protocols in Molecular Biology (John Wiley & Sons, Inc).

The present invention provides a method of functional analysis of a gene or a protein associated with an infused nucleic acid, characterized by using the above-described cell of the present invention or the above-described RNA or protein of the present invention.

The method of nucleic acid infusion of the present invention allows every nucleic acid infusion as described above, and can be used particularly preferably in the construction of a knockdown cell, which inhibits the expression and function of a target gene.

Examples of the functional analysis the uses the knockdown cell include analysis in which an antisense nucleic acid, small interfering RNA or a decoy nucleic acid is infused into the cell and the production or function of a target gene or a protein encoded by the gene is inhibited to examine the function of the gene in the cell.

It is preferred that the method of functional analysis of the present invention should conducted using a cell obtained by infusing a nucleic acid into a cell that is hardly infused with a nucleic acid heretofore, that is, a macrophage-like cell line RAW264.7 cell, a hybridoma DO11.10 cell, a primary cultured hepatocyte, a primary cultured myoblast, a primary cultured muscle cell, a primary cultured adipocyte precursor cell, a primary cultured adipocyte or a primary cultured neuron by the method of nucleic acid infusion of the present invention, or using RNA or a protein derived from the cell.

### EXAMPLES

Hereinafter, the present invention will be described more specifically with reference to Examples. However, the present invention is not intended to be limited to these Examples by any means.

### Example 1

### Infusion of oligonucleotide into murine macrophage-like cell line RAW264.7 cell using method of nucleic acid infusion of the present invention and conventional methods

Murine macrophage-like cell line RAW264.7 cells were used to compare a method of nucleic acid infusion of the present invention that utilized osmotic shock (hereinafter, also referred to as an osmotic transfection method) with conventional methods.

### 1-1) Infusion of oligonucleotide into murine macrophage-like cell line RAW264.7 cell by method of nucleic acid infusion of the present invention

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO.: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at 37°C for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation for 5 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 1-2) Infusion of oligonucleotide into murine macrophage-like cell line RAW264.7 cell with Oligofectamine

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Infusion of oligonucleotide

An oligonucleotide/Oligofectamine mixture solution was prepared according to Invitrogen's recommendation manual. Specifically, 9 µl of Oligofectamine was suspended in 150 µl of OPTI-MEMI (Invitrogen) and left undisturbed at room temperature for 5 minutes. The resulting mixture was mixed with an equal amount of diluted oligonucleotide solution (a mixture solution of 9 µl of 20 µM FITC-labeled oligonucleotide and 150 µl of OPTI-MEMI) and left at room temperature for 20 minutes to obtain an oligonucleotide/Oligofectamine mixture solution. Then, the above-described cells prepared in the 24-well plate were washed with OPTI-MEMI to remove the culture supernatant. To the cells, 318 µl of the oligonucleotide/Oligofectamine mixture solution was gently added and left undisturbed at 37°C for 6 hours under 5% CO₂ to remove the resulting supernatant. The mixture was supplemented with 3 ml of the growth medium RPMI1640 (Invitrogen) containing 10% (v/v) fetal calf serum. After infusion at 37°C under 5% CO₂, culture was conducted for 24 hours.

### 3. Observation of infusion efficiency

Infusion efficiency was observed using the CCD camera as in the above-described Example 1-1).

### 1-3) Infusion of oligonucleotide into murine macrophage-like cell line RAW264.7 cell with polyethylenimine

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Infusion of oligonucleotide

Polyethylenimine used was jetPEI (PolyPlus). An oligonucleotide/polyethylenimine mixture solution was prepared according to PolyPlus's recommendation manual. Specifically, 8 µl of 7.5 mM jetPEI was suspended in 192 µl of 150 mM NaCl aqueous solution (Nacalai Tesque) and mixed with an equal amount of diluted oligonucleotide solution (40 µl of 20 µM FITC-labeled oligonucleotide and 160 µl of 150 mM NaCl aqueous solution) and left at room temperature for 15 minutes to obtain an oligonucleotide/polyethylenimine mixture solution. Then, 400 µl of the oligonucleotide/polyethylenimine mixture solution was gently added to the above-described cells prepared in the 24-well plate. After infusion at 37°C for 24 hours under 5% CO₂, culture was conducted for 24 hours.

### 3. Observation of infusion efficiency

Infusion efficiency was observed using the CCD camera as in the above-described Example 1-1).

### 1-4) Comparison of results of oligonucleotide infusion among method of nucleic acid infusion of the present invention, Oligofectamine method and polyethylenimine method

The morphology of the cells, damage to the cells and infusion efficiency after 24 hours of FITC-labeled oligonucleotide infusion were compared among the above-described method of nucleic acid infusion of the present invention, Oligofectamine method and polyethylenimine method. The result is shown in FIG. 1.

As a result, in the method of nucleic acid infusion of the present invention, the morphological change and damage of the cells were not observed, and oligonucleotide infusion was observed in 80% or more of the cells in the visual field. On the other hand, in the Oligofectamine method and the polyethylenimine method, fluorescent oligonucleotide infusion was observed in almost all of the cells in the visual field. However, decrease in the number of cells due to cell exfoliation during procedures for infusion was observed, while the morphological change of all of the cells in the bright field was observed and the great majority of the cells burst and died. These results have shown that unlike the Oligofectamine method and the polyethylenimine method, the method of nucleic acid infusion of the present invention can infuse oligonucleotide without causing the morphological change and damage of cells.

### Example 2

### Infusion of TLR4 siRNA into RAW264.7 cell by method of nucleic acid infusion of the present invention and measurement of siRNA effect by quantitative PCR

### 1. Infusion of TLR4 siRNA into RAW264.7 cell by method of nucleic acid infusion of the present invention

TLR4 siRNA (double-stranded RNA with a sense sequence 5'-AUCCACAAGUCAAUCUCUCUU-3'; SEQ ID NO: 2 and an antisense sequence 5'-GAGAGAUUGACUUGUGGAUUU-3'; SEQ ID NO: 3) was synthesized by Proligo. Negative Control #1 siRNA was purchased from Ambion. The infusion of TLR4 siRNA and Negative Control #1 siRNA into RAW264.7 cells by the method of nucleic acid infusion of the present invention was performed by the same approach as the above-described Example 1-1) after a hypertonic solution containing 10 µM TLR4 siRNA or Negative Control #1 siRNA was prepared in the same way as Example 1-1).

### 2. Collection of total RNA from infused cell and quantitative PCR

After 24 hours of infusion, total RNA was collected from the cells using an RNeasy kit (Qiagen). Primers specific to TLR4 mRNA (Forward Primer: 5'-GGATGATGCCTCCCTGGC-3' (SEQ ID NO: 11) and Reverse Primer: 5'-GGGATTCAAGCTTCCTGGTG-3' (SEQ ID NO: 12)) and primers specific to GAPDH mRNA that served as a normalization control (Forward Primer: 5'-AACTCGGCCCCCAACACT-3' (SEQ ID NO: 13) and Reverse Primer: 5'-TAGGCCCCTCCTGTTATTATGG -3' (SEQ ID NO: 14)) were used to perform quantitative PCR as described below.

At first, TaqMan Reverse Transcription Reagents (Applied Biosystems) were used to synthesize cDNA with 1 µg of total RNA as a template according to the kit's protocol. The obtained cDNA derived from 50 ng of total RNA, which was used as a template, was mixed with a specific primer set for detection and SYBR Green PCR Master Mix and RT-PCR Kit (Applied Biosystems) according to the kit's protocol to perform measurement with a real-time PCR system ABI7700 (Applied Biosystems). The rate of decrease in TLR4 mRNA was calculated with the expression level of GAPDH regarded as being equal between Negative Control #1 siRNA- and TLR4 siRNA-infused groups. The result is shown in FIG. 2.

As can be seen from the result, in the TLR4 siRNA-infused group, TLR4 mRNA disappeared 83% as compared with the Negative Control #1 siRNA-infused group.

These results have shown that the method of nucleic acid infusion of the present invention can infuse not only fluorescently labeled oligonucleotide but also siRNA and that infused siRNA allows mRNA in cells to disappear with high efficiency.

### Example 3

### Infusion of TLR4 siRNA into RAW264.7 cell by method of nucleic acid infusion of the present invention and bioassay using knockdown cell

TLR4 is a receptor of LPS (Calbiochem) and GLA-60 (WAKO CHEMICAL), and RAW264.7 cells are known to induce TNFα production by the stimulation of the cells with these compounds. Therefore, TLR4 siRNA was infused into RAW264.7 cells as described in Example 2. After 24 hours, the cells were scraped with a scraper and suspended in a growth medium. The resulting cells were seeded at 10000 cells/well to a 96-well plate and cultured at 37°C under 5% CO₂. After 48 hours of the infusion of siRNA, the resulting culture supernatant was removed. Stimulation without or with 10 µg/ml GLA-60 or 10 ng/ml LPS for 8 hours was performed to collect the culture supernatant. Instead of TLR4 siRNA, Negative Control #1 siRNA (Sequitur) was infused as a control under the same conditions. After 48 hours, the resulting culture supernatant was removed. Stimulation without or with 10 µg/ml GLA-60 or 10 ng/ml LPS for 8 hours was performed to collect the culture supernatant.

TNFα contained in the culture supernatants of the TLR4 siRNA-infused cells and the control cells was quantified with a Mouse TNF-α AN'ALYZA ELISA kit (Cosmo Bio). The result is shown in FIG. 3. As can be seen from the result, in the TLR4 siRNA-infused cells, TNFα production decreased 45% by stimulation with LPS and 66% by stimulation with GLA-60 as compared with the control cells.

These results have shown that the induction of TNFα production in response to stimulation with LPS or GLA-60 is observed in cells infused with Negative Control siRNA by the method of nucleic acid infusion of the present invention as with usual cells whereas the induction of TNFα production in response to stimulation with these compounds is significantly inhibited in TLR4 siRNA-infused cells. Accordingly, these results have indicated that siRNA infusion by the method of nucleic acid infusion of the present invention inhibits cell function, and more specifically, have indicated that the method of the present invention is useful for gene functional analysis such as the identification of a receptor-ligand pair.

### Example 4

### Infusion of oligonucleotide into primary cultured rat hepatocyte by method of nucleic acid infusion of the present invention

### 1. Preparation of cell

A primary cultured rat hepatocyte was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the hepatocytes was removed, and 400 µl of the hypertonic solution containing oligonucleotide was added to the cells. Following incubation at room temperature for 3 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 5 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

Fluorescent oligonucleotide infusion was observed in almost all of the cells in the visual field. Moreover, morphological change and cell death were not observed in the cells in the bright field.

### Example 5

### Infusion of oligonucleotide into primary cultured rat myoblast by method of nucleic acid infusion of the present invention

### 1. Preparation of cell

A primary cultured rat myoblast was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the myoblasts was removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 5 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

Fluorescent oligonucleotide infusion was observed in almost all of the cells in the visual field. Moreover, morphological change and cell death were not observed in the cells in the bright field.

### Example 6

### Infusion of oligonucleotide into primar cultured rat muscle cell by method of nucleic acid infusion of the present invention

### 1. Préparation of cell

A primary cultured rat myoblast was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured overnight under conditions of 37°C and 5% CO₂. Then, the resulting culture supernatant was removed, and the medium was replaced with an accompanying myoblast differentiation medium. The cells were cultured for 5 days to obtain differentiated muscle cells.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the differentiated muscle cells was removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 5 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

Fluorescent oligonucleotide infusion was observed in almost all of the cells in the visual field. Moreover, morphological change and cell death were not observed in the cells in the bright field.

### Example 7

### Infusion of oligonucleotide into primary cultured rat adipocyte by method of nucleic acid infusion of the present invention

### 1. Preparation of cell

A primary cultured rat white adipocyte precursor cell was purchased from Hokudo. Specifically, the cells were received with them seeded at a density of 100000 cell/well in a 24-well plate (Nalge Nunc). Immediately after the receipt of the cells, the original medium was replaced with an accompanying differentiation medium, and the cells were cultured to obtain differentiated white adipocytes.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well late

The culture supernatant of the differentiated adipocytes was removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

Fluorescent oligonucleotide infusion was observed in almost all of the cells in the visual field. Moreover, morphological change and cell death were not observed in the cells in the bright field.

### Example 8

### Infusion of oligonucleotide into primary cultured rat neuron by method of nucleic acid infusion of the present invention

### 1. Preparation of cell

A primary cultured rat neuron was purchased from Sumitomo Bakelite. Specifically, the cells were seeded at a density of 100000 cell/well in an accompanying 24-well plate (Sumitomo Bakelite). After replacement with an accompanying medium, the cells were cultured to obtain neurons.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the neurons was removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

Fluorescent oligonucleotide infusion was observed in almost all of the cells in the visual field. Moreover, morphological change and cell death were not observed in the cells in the bright field.

### Example 9

### Infusion of oligonucleotide into primary cultured rat adipocyte precursor cell using method of nucleic acid infusion of the present invention and conventional methods

Primary cultured rat adipocyte precursor cells were used to compare the method of nucleic acid infusion of the present invention with conventional methods.

### 9-1) Infusion of oligonucleotide into primary cultured rat adipocyte precursor cell by method of nucleic acid infusion of the present invention

### 1. Preparation of cell

A primary cultured rat white adipocyte precursor cell was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the white adipocyte precursor cells was removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 9-2) Infusion of oligonucleotide into primary cultured rat adipocyte precursor cell with Oligofectamine

### 1. Preparation of cell

A primary cultured rat white adipocyte precursor cell was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured.

### 2. Infusion of oligonucleotide

An oligonucleotide/Oligofectamine mixture solution was prepared according to Invitrogen's recommendation manual. Specifically, 9 µl of Oligofectamine was suspended in 150 µl of OPTI-MEMI (Invitrogen) and left undisturbed at room temperature for 5 minutes. The resulting mixture was mixed with an equal amount of diluted oligonucleotide solution (a mixture solution of 9 µl of 20 µM FITC-labeled oligonucleotide and 150 µl of OPTI-MEMI) and left at room temperature for 20 minutes to obtain an oligonucleotide/Oligofectamine mixture solution. Then, the above-described cells prepared in the 24-well plate were washed with OPTI-MEMI to remove the culture supernatant. To the cells, 318 µl of the oligonucleotide/Oligofectamine mixture solution was gently added and left undisturbed at 37°C for 6 hours under 5%CO₂ to remove the resulting supernatant. The mixture was supplemented with 3 ml of the growth medium. After infusion at 37°C under 5% CO₂, culture was conducted for 24 hours.

### 3. Observation of infusion efficiency

Infusion efficiency was observed using the CCD camera as in the above-described Example 9-1).

### 9-3) Infusion of oligonucleotide into primary cultured rat adipocyte precursor cell with Lipofectamine 2000

### 1. Preparation of cell

A primary cultured rat white adipocyte precursor cell was purchased from Hokudo. Specifically, the cells were received with them seeded at a density of 100000 cell/well in a 24-well plate (Nalge Nunc). Immediately after the receipt of the cells, the original medium was replaced with an accompanying growth medium, and the cells were cultured.

### 2. Infusion of oligonucleotide

An oligonucleotide/Lipofectamine 2000 mixture solution was prepared according to Invitrogen's recommendation manual. Specifically, 3 µl of Lipofectamine 2000 was suspended in 150 µl of OPTI-MEMI (Invitrogen) and left undisturbed at room temperature for 5 minutes. The resulting mixture was mixed with an equal amount of diluted oligonucleotide solution (a mixture solution of 3 µl of 20 µM FITC-labeled oligonucleotide and 150 µl of OPTI-MEMI) and left at room temperature for 20 minutes to obtain an oligonucleotide/Lipofectamine 2000 mixture solution. Then, the above-described cells prepared in the 24-well plate were washed with OPTI-MEMI to remove the culture supernatant. To the cells, 306 µl of the oligonucleotide/Lipofectamine 2000 mixture solution was gently added and left undisturbed at 37°C for 6 hours under 5%CO₂ to remove the resulting supernatant. The mixture was supplemented with 3 ml of the growth medium. After infusion at 37°C under 5% CO₂, culture was conducted for 24 hours.

### 3. Observation of infusion efficiency

Infusion efficiency was observed using the CCD camera as in the above-described Example 9-1).

### 9-4) Comparison of results of oligonucleotide infusion into primary cultured rat adipocyte precursor cell among method of nucleic acid infusion of the present invention, Oligofectamine method and Lipofectamine 2000 method

The morphology of the cells, damage to the cells and infusion efficiency after 24 hours of FITC-labeled oligonucleotide infusion were compared among the above-described method of nucleic acid infusion of the present invention, Oligofectamine method and Lipofectamine method.

As a result, in the method of nucleic acid infusion of the present invention, the morphological change and damage of the cells were not observed, and oligonucleotide infusion was observed in approximately 80% of the cells in the visual field. On the other hand, in the Oligofectamine method and the Lipofectamine method, FITC-labeled oligonucleotide infusion was not observed in the great majority of the cells in the visual field. These results have shown that unlike the Oligofectamine method and the Lipofectamine method, the method of nucleic acid infusion of the present invention can also infuse oligonucleotide into primary cultured rat adipocyte precursor cells without causing the morphological change and damage of cells.

### Example 10

### Investigation on effect of sucrose concentration in hypertonic solution on infusion efficiency

While the composition of the hypertonic solution was the same as Example 1-1) except for sucrose, change in infusion efficiency was examined at varying sucrose concentrations: 0.5 M, 0.75M and 1 M.

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 0.5 M, 0.75M or 1 M sucrose, 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

When the hypertonic solution containing 0.75 M or 1 M sucrose was used, fluorescent oligonucleotide infusion was observed in almost all of the cells in the visual field. When the hypertonic solution containing 0.5 M sucrose was used, fluorescent oligonucleotide infusion into the cells was observed although the number of cells infused with oligonucleotide was slightly lower than those for the hypertonic solution containing 0.75 M or 1 M sucrose. When the hypertonic solution of any of the sucrose concentrations was used, morphological change and cell death were not observed in the cells in the bright field. These results have shown that a more preferable sucrose concentration in a hypertonic solution is 0.5 M to 1 M.

### Example 11

### Investigation on effect of type of sugar in hypertonic solution on infusion efficiency

While the molarity of the solute in the hypertonic solution was the same as Example 1-1), change in infusion efficiency was examined with various types of sugars: sucrose, mannitol, sorbitol and xylitol.

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sugar (sucrose, mannitol, sorbitol or xylitol), 10% (w/v) polyethylene glycol 1000, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

No difference in infusion efficiency was observed among the sugars, sucrose, mannitol, sorbitol and xylitol. These results have shown that any sugar can be used as a solute in a hypertonic solution.

### Example 12

### Investigation on effect of type of PEG in hypertonic solution on infusion efficiency

While the molarity of the solute in the hypertonic solution was the same as Example 1-1), change in infusion efficiency was examined with various types of PEGs: PEG200, PEG300, PEG400, PEG600, PEG1000, PEG1500, PEG1540 and PEG2000.

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% C0₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) (=100 mM) polyethylene glycol 1000 or equal molarity of PEG (PEG200, PEG300, PEG400, PEG600, PEG1000, PEG1500, PEG1540 or PEG2000) , RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

No difference in infusion efficiency was observed among the degrees of polymerization (average molecular weights) of PEGs. These results have shown that PEG with any degree of polymerization (average molecular weight) can be used as a solute in a hypertonic solution.

### Example 13

### Investigation on effect of substitution of glycerol for PEG in hypertonic solution on infusion efficiency

While the molarity of the solute in the hypertonic solution was the same as Example 1-1), change in infusion efficiency was examined with equal molarity of glycerol substituted for 10% (w/v) (=100 mM) PEG1000.

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose, 10% (w/v) (=100 mM) polyethylene glycol 1000 or 100 mM glycerol, RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

No difference in infusion efficiency was observed between PEG and glycerol used. These results have shown that not only PEG but also glycerol can be used as a solute in a hypertonic solution.

### Example 14

### Investigation on effect of substitution of equal molarity of sucrose or sugar alcohol for PEG and sucrose in hypertonic solution of Example 1-1) on infusion efficiency

While the molarity of the solute in the hypertonic solution was the same as Example 1-1), change in infusion efficiency was examined with equal molarity of sucrose, sorbitol, mannitol or xylitol substituted for 10% (w/v) (=100 mM) PEG1000 and 1 M sucrose.

### 1. Preparation of cell

RAW264.7 cells (ATCC No. TIB-71) were suspended in RPMI1640 (Invitrogen) containing 10% (v/v) fetal calf serum. The resulting cells were seeded at a density of 100000 cells/well into a 24-well plate (Nalge Nunc) and cultured overnight under conditions of 37°C and 5% CO₂.

### 2. Preparation of hypertonic solution containing oligonucleotide

A hypertonic solution containing oligonucleotide was prepared to have the following composition: 10 µM FITC-labeled oligonucleotide (20-mer; 5'-GGA ACT TCC CTA AAG GGA GG-3'; SEQ ID NO: 1), 1 M sucrose and 10% (w/v) (=100 mM) PEG1000 or 1.1 M sugar (sucrose, sorbitol, mannitol or xylitol), RPMI1640 (Invitrogen), 5% (v/v) fetal bovine serum, and 10 mM HEPES buffer solution (pH 7.4).

### 3. Preparation of hypotonic solution

A hypotonic solution was prepared to be a mixture solution of a RPMI1640 medium and DEPC-treated water (6:4).

### 4. Growth medium

RPMI1640 (Invitrogen) was supplemented with 10% (v/v) fetal bovine serum and 60 mg/L kanamycin sulfate to prepare a growth medium.

### 5. Infusion in 24-well plate

The RAW264.7 cells were seeded at 100000 cells/well and cultured overnight. The resulting culture supernatant was then removed, and 400 µl of the hypertonic solution containing oligonucleotide was added to the cells. Following incubation at room temperature for 10 minutes, the hypertonic solution containing oligonucleotide was removed. Immediately after the addition of 3 ml of the hypotonic solution, the hypotonic solution was removed. Again, 3 ml of the hypotonic solution was added. Following incubation at room temperature for 2 minutes, the hypotonic solution was removed, and 3 ml of the growth medium was added.

### 6. Observation of infusion efficiency

Infusion efficiency was observed by using a differential interference microscope Axiovert 135 (Carl Zeiss) equipped with a 20x objective and a super high-sensitivity CCD camera (Hamamatsu Photonics) to measure excitation light from a fluorescence microscope system: a fluorescence microscopy apparatus for Axiovert 135 with an ultraviolet light source (Carl Zeiss), which passed through a FITC band pass filter. After 24 hours of infusion, FITC fluorescence was photographed with an exposure for 2 seconds by the above-described fluorescence microscope system. The morphology of the cells was photographed with an exposure for 0.03 seconds under conditions of a visible light source and no filter by the same system.

### 7. Result

As can be seen from the result, the use of a variety of simple sugar substances (sucrose, sorbitol, mannitol and xylitol) allowed oligonucleotide infusion as with the use of a mixture of PEG and sucrose when the simple sugar substance was used at the same molarity (1.1 M) as that of the mixture of PEG and sucrose. These results have shown that not only a mixture of PEG and sucrose but also each simple ingredient substance can be used as a solute in a hypertonic solution.

### Example 15

### Infusion of oligonucleotide into murine macrophage-like cell line RAW264.7 cell usin method of nucleic acid infusion of the present invention

For examining infusion efficiency and cytotoxicity by the method of nucleic acid infusion of the present invention, a murine macrophage-like cell line RAW264.7 cell was used to examine, by flow cytometry, the effect of FITC-labeled nucleic acid infusion and the presence or absence of cytotoxicity caused by infusion.

### 15-1) Infusion of FITC-labeled oligonucleotide into murine macrophage-like cell line RAW264.7 cell by method of nucleic acid infusion of the present invention

### 1. Preparation of cell

RAW264.7 cells were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 1.4 M sucrose, 100 mM (10%) PEG1000, RPMI1640 (Invitrogen), 5% (v/v) fetal bovine serum, and 10 mM HEPES buffer solution (pH 7.4).

### 3. Preparation of FITC-labeled oligonucleotide solution

A FITC-labeled oligonucleotide solution was prepared in the same way as Example 1.

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 500000 RAW264.7 cells were collected and centrifuged at 2000 rpm for 5 minutes with a high-speed tabletop centrifuge (Tomy Seiko) to remove the resulting culture supernatant. To the tube, 10 µl of 1 mM FITC-labeled oligonucleotide solution and 190 µl of the hypertonic solution were added. Following incubation at 37°C for 10 minutes, 1.1 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI).

### 15-2) Measurement of effect of FITC-labeled oligonucleotide infusion

A flow cytometer EPICS-XL II system was used to examine the effect of oligonucleotide infusion with the amount of fluorescence emitted from fluorescein as an indicator.

After 24 hours of infusion, the cells were collected and centrifuged at 2000 rpm for 5 minutes with a high-speed tabletop centrifuge (Tomy Seiko) to remove the resulting culture supernatant. The resulting cells were resuspended in 1 ml of the growth medium. The fluorescence intensity of the fluorescein in the obtained cells was measured using the EPICS-XL II system (Beckman Coulter). Fig 4 (upper diagram) shows a result for control cells obtained by using an equal amount of DEPC-treated water instead of FITC-labeled oligonucleotide and the cells infused with FITC-labeled oligonucleotide by the method of infusion of the present invention.

As a result, it has been shown that the use of the method of infusion of the present invention can efficiently infuse oligonucleotide into murine macrophage-like cell line RAW264.7 cells.

### 15-3) Measurement of cytotoxicity

A flow cytometer EPICS-XL II system was used to examine cytotoxicity during infusion treatment with the amount of fluorescence emitted from 7-actinomycin D (7AAD), a reagent for staining dead cells, as an indicator.

After 24 hours of infusion treatment, the cells were collected and centrifuged at 2000 rpm for 5 minutes with a high-speed tabletop centrifuge (Tomy Seiko) to remove the resulting culture supernatant. The resulting cells were resuspended in 1 ml of the growth medium. After 10 µl of a 7AAD solution (Beckman Coulter) was added and incubated at room temperature for 10 minutes, the fluorescence intensity of the 7AAD in the obtained cells was measured using the EPICS-XL II system (Beckman Coulter). Fig 4 (lower diagram) shows a result for control cells treated in the same way using the growth medium instead of the hypertonic and hypotonic solutions and the cells subjected to infusion treatment by the method of infusion of the present invention.

As a result, it has been shown that the infusion treatment of the present invention does not exhibit cytotoxicity.

These results have demonstrated that the method of infusion of the present invention can efficiently infuse a nucleic acid and does not cause cytotoxicity.

### Example 16

### Infusion of siRNA into murine macrophage-like cell line RAW264.7 cell using method of nucleic acid infusion of the present invention

Murine macrophage cell line RAW264.7 cells were used to investigate the effect of types of oligosaccharide and polyhydric alcohol contained in the hypertonic solution on siRNA infusion into cells. 16-1) Infusion of siRNA into murine macrophage-like cell line RAW264.7 cell by method of nucleic acid infusion of the present invention

### 1. Preparation of cell

RAW264.7 cells (ATCC No. TIB-71) were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 1.4 M oligosaccharide or polyhydric alcohol, RPMI1640 (Invitrogen), 5% (v/v) fetal bovine serum, and 10 mM HEPES buffer solution (pH 7.4). Oligosaccharide used was sucrose (disaccharide, Sigma), and polyhydric alcohol used was sorbitol (monosaccharide alcohol, Sigma), mannitol (monosaccharide alcohol, Sigma), xylitol (monosaccharide alcohol, Sigma), maltitol (disaccharide alcohol, Sigma) or glycerol (triol, Nacalai Tesque).

### 3. Preparation of siRNA solution

siRNA was purchased from Proligo Japan as a freeze-dried product after the annealing of double strands. This siRNA was dissolved in DEPC-treated water (Nacalai Tesque) to adjust its concentration to 1 mM. The siRNA used was as follows: 1 mM Negative Control siRNA (21-mer sense strand; 5'-CAAGCUGACCCUGAAGUUCAU-3'; SEQ ID NO: 4 and 21-mer antisense strand; 5'-GAACUUCAGGGUCAGCUUGUU-3'; SEQ ID NO: 5, Proligo Japan) and 1 mM GAPDH siRNA (21-mer sense strand; 5'-CCACGAGAAAUAUGACAACUC-3; SEQ ID NO: 6 and 21-mer antisense strand; 5'-GUUGUCAUAUUUCUCGUGGUU-3'; SEQ ID NO: 7).

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 100000 RAW264.7 cells were collected and centrifuged at 2000 rpm for 5 minutes with a high-speed tabletop centrifuge (Tomy Seiko) to remove the resulting culture supernatant. To the tube, 10 µl of 1 mM siRNA solution and 190 µl of each hypertonic solution were added. Following incubation at 37°C for 10 minutes, 1.1 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI).

### 16-2) Measurement of effect on siRNA infusion

The effect of types of oligosaccharide and polyhydric alcohol contained in the hypertonic solution on siRNA infusion into cells was examined with the efficiency of GAPDH siRNA-mediated knockdown of endogenous GAPDH mRNA as an indicator.

After 24 hours of infusion, total RNA was collected from the cells using an RNeasy kit (Qiagen). Primers specific to GAPDH mRNA (Forward Primer: 5'-TCACCACCATGGAGAAGGC-3' (SEQ ID NO: 15) and Reverse Primer: 5'-CTAAGCAGTTGGTGGTGCAG-3' (SEQ ID NO: 16)) and primers specific to cyclophilin mRNA that served as a normalization control (Forward Primer: 5'-GTCAACCCCACCGTGTTCT-3' (SEQ ID NO: 17) and Reverse Primer: 5'-GAGGTACCTGCTGTCTTTGG-3' (SEQ ID NO: 18)) were used to perform quantitative PCR in the same way as Example 2. The rate of decrease in GAPDH mRNA was calculated with the expression level of cyclophilin regarded as being equal between Negative Control siRNA-and GAPDH siRNA-infused groups. The result is shown in FIG. 5.

As can be seen from the result, in the GAPDH siRNA-infused group, GAPDH mRNA disappeared 80.2% for the group with the hypertonic solution containing sucrose, 68.5% for the group with the hypertonic solution containing sorbitol, 75.6% for the group with the hypertonic solution containing mannitol, 74.7% for the group with the hypertonic solution containing xylitol, 69.8% for the group with the hypertonic solution containing maltitol and 38.2% for the group with the hypertonic solution containing glycerol as compared with the Negative Control siRNA-infused group.

These results have shown that the use of any oligosaccharide and polyhydric alcohol as a solute in a hypertonic solution also allows the efficient infusion of siRNA as with FITC-labeled oligonucleotide and that the infused siRNA exhibits knockdown activity. Because the individual use of a variety of oligosaccharides and polyhydric alcohols as a solute in addition to the combined use of oligosaccharide and PEG allowed the efficient siRNA infusion, it has been confirmed that the effect of infusion depends on the contact between cells and a hypertonic solution.

### Example 17

### Infusion of siRNA into murine macrophage-like cell line RAW264.7 cell using method of nucleic acid infusion of the present invention

Murine macrophage cell line RAW264.7 cells were used to investigate the effect of a combination of oligosaccharide and polyhydric alcohol contained in the hypertonic solution on siRNA infusion into cells. 17-1) Infusion of siRNA into murine macrophage-like cell line RAW264.7 cell by method of nucleic acid infusion of the present invention

### 1. Preparation of cell

RAW264.7 cells (ATCC No. TIB-71) were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 1.4 M oligosaccharide, 100 mM polyhydric alcohol, RPMI1640 (Invitrogen), 5% (v/v) fetal bovine serum, and 10 mM HEPES buffer solution (pH 7.4). Oligosaccharide used was sucrose or sorbitol, and polyhydric alcohol used was polyethylene glycol 200 (PEG200), PEG300, PEG400, PEG600, PEG1000, PEG1500, PEG1540 or PEG2000. Sucrose and sorbitol were purchased from Sigma, and PEGs were purchased from Nacalai Tesque.

### 3. Preparation of siRNA solution

siRNA was purchased from Proligo Japan as a freeze-dried product after the annealing of double strands. This siRNA was dissolved in DEPC-treated water (Nacalai Tesque) to adjust its concentration to 1 mM. The siRNA used was as follows: 1 mM Negative Control siRNA (21-mer sense strand; 5'-CAAGCUGACCCUGAAGUUCAU-3'; SEQ ID NO: 4 and 21-mer antisense strand; 5'-GAACUUCAGGGUCAGCUUGUU-3'; SEQ ID NO: 5, Proligo Japan) and 1 mM GAPDH siRNA (21-mer sense strand; 5'-CCACGAGAAAUAUGACAACUC-3; SEQ ID NO: 6 and 21-mer antisense strand; 5'-GUUGUCAUAUUUCUCGUGGUU-3'; SEQ ID NO: 7).

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 100000 RAW264.7 cells were collected and centrifuged at 2000 rpm for 5 minutes with a high-speed tabletop centrifuge (Tomy Seiko) to remove the resulting culture supernatant. To the tube, 10 µl of 1 mM siRNA solution and 190 µl of each hypertonic solution were added. Following incubation at 37°C for 10 minutes, 1.1 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI).

### 17-2) Measurement of effect on siRNA infusion

The effect of a combination of oligosaccharide and polyhydric alcohol contained in the hypertonic solution on siRNA infusion into cells was examined with the efficiency of GAPDH siRNA-mediated knockdown of endogenous GAPDH mRNA as an indicator.

After 24 hours of infusion, total RNA was collected from the cells using an RNeasy kit (Qiagen). Primers specific to GAPDH mRNA (Forward Primer: 5'-TCACCACCATGGAGAAGGC-3' (SEQ ID NO: 15) and Reverse Primer: 5'-CTAAGCAGTTGGTGGTGCAG-3' (SEQ ID NO: 16)) and primers specific to cyclophilin mRNA that served as a normalization control (Forward Primer: 5'-GTCAACCCCACCGTGTTCT-3' (SEQ ID NO: 17) and Reverse Primer: 5'-GAGGTACCTGCTGTCTTTGG-3' (SEQ ID NO: 18)) were used to perform quantitative PCR in the same way as Example 2. The rate of decrease in GAPDH mRNA was calculated with the expression level of cyclophilin regarded as being equal between Negative Control siRNA-and GAPDH siRNA-infused groups. A result for the combination of sucrose and a variety of PEGs is shown in FIG. 6, and a result for the combination of sorbitol and a variety of PEGs is shown in FIG. 7.

As can be seen from the result, in the combination of sucrose and a variety of PEGs, GAPDH mRNA disappeared 69.2% for the group with the hypertonic solution containing sucrose and PEG200, 77.9% for the group with the hypertonic solution containing sucrose and PEG300, 69.1% for the group with the hypertonic solution containing sucrose and PEG400, 72.9% for the group with the hypertonic solution containing sucrose and PEG600, 68.7% for the group with the hypertonic solution containing sucrose and PEG1000 and 73.3% for the group with the hypertonic solution containing sucrose and PEG1500. As can also be seen from the result, in the combination of sorbitol and a variety of PEGs, GAPDH mRNA disappeared 65.0% for the group with the hypertonic solution containing sorbitol and PEG200, 62.9% for the group with the hypertonic solution containing sorbitol and PEG300, 49.6% for the group with the hypertonic solution containing sorbitol and PEG400, 41.8% for the group with the hypertonic solution containing sorbitol and PEG600, 70.5% for the group with the hypertonic solution containing sorbitol and PEG1000, 57.7% for the group with the hypertonic solution containing sorbitol and PEG1500, 37.8% for the group with the hypertonic solution containing sorbitol and PEG1540 and 45.4% for the group with the hypertonic solution containing sorbitol and PEG2000.

These results have shown that the use of a combination of oligosaccharide and polyhydric alcohol as a solute in a hypertonic solution also allows the efficient infusion of siRNA as with FITC-labeled oligonucleotide and that the infused siRNA exhibits knockdown activity. The use of a combination of sugar alcohol and PEGs as a solute in addition to a combination of oligosaccharide and PEGs allowed the efficient siRNA infusion, and the infused siRNA exhibited knockdown activity. Therefore, it has been confirmed that various combinations of oligosaccharide and polyhydric alcohol can be used as a solute in a hypertonic solution.

### Example 18

### Infusion of morpholino antisense oligonucleotide into murine T cell-like cell line DO11.10 cell using method of nucleic acid infusion of the present invention

For determining whether or not the method of nucleic acid infusion of the present invention required the electric charge of a nucleic acid, murine T cell-like cell line DO11.10 cells were used to examine the effect of infusion of morpholino antisense oligonucleotide, a nucleic acid analog with no electric charge.

### 18-1) Infusion of morpholino antisense oligonucleotide into murine hybridoma DO11.10 cell by method of nucleic acid infusion of the present invention

### 1. Preparation of cell

DO11.10 cells were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 2.0 M sucrose, 100 mM PEG1000, RPMI1640 (Invitrogen), 5% (v/v) fetal bovine serum, and 10 mM HEPES buffer solution (pH 7.4).

### 3. Preparation of morpholino antisense oligonucleotide solution

Fluorescein-labeled morpholino antisense oligonucleotide was purchased from Funakoshi as a freeze-dried product. This morpholino antisense oligonucleotide was dissolved in DEPC-treated water (Nacalai Tesque) to adjust its concentration to 1 mM. The fluorescein-labeled morpholino antisense oligonucleotide used was as follows: 1 mM morpholino antisense (25-mer; 5'-CCTCTTACCTCAGTTACAATTTATA-3'; SEQ ID NO: 8).

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 500000 DO11.10 cells were collected and centrifuged at 2000 rpm for 5 minutes with a high-speed tabletop centrifuge (Tomy Seiko) to remove the resulting culture supernatant. To the tube, 10 µl of 1 mM morpholino antisense oligonucleotide solution and 190 µl of the hypertonic solution were added. Following incubation at 37°C for 10 minutes, 1.1 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI).

### 18-2) Measurement of effect of morpholino antisense oligonucleotide infusion

A flow cytometer EPICS-XL II system was used to examine the effect of morpholino antisense oligonucleotide infusion with the amount of fluorescence emitted from fluorescein as an indicator.

After 24 hours of infusion, the cells were collected and centrifuged at 2000 rpm for 5 minutes with a high-speed tabletop centrifuge (Tomy Seiko) to remove the resulting culture supernatant. The resulting cells were resuspended in 1 ml of the growth medium. The fluorescence intensity of the fluorescein in the obtained cells was measured using the EPICS-XL II system (Beckman Coulter). Fig 8 (upper diagram) shows a result for control cells obtained by using an equal amount of DEPC-treated water instead of morpholino antisense oligonucleotide and the cells infused with morpholino antisense oligonucleotide by the method of infusion of the present invention. FIG. 8 (lower diagram) shows a result of infusing FITC-labeled phosphorothioate oligodeoxynucleotide, a nucleic acid analog with an electric charge, instead of morpholino antisense oligonucleotide under the same conditions

As can be seen from the result, the use of the method of infusion of the present invention allowed the efficient infusion of, into cells, not only a nucleic acid with an electric charge but also morpholino antisense oligonucleotide, a nucleic acid analog with no electric charge.

These results have shown that the effect of the method of infusion of the present invention dose not depend on the electric charge of a nucleic acid.

### Example 19

### Repetitive infusion of siRNA into murine hybridoma DO11.10 cell using method of nucleic acid infusion of the present invention

Murine hybridoma DO11.10 cells were used to examine the effect of GAPDH siRNA repetitive infusion. 19-1) Infusion of siRNA into murine hybridoma DO11.10 cell by method of nucleic acid infusion of the present invention

### 1. Preparation of cell

DO11.10 cells were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 2.0 M sucrose, 100 mM PEG1000, RPMI1640 (Invitrogen), 5% (v/v) fetal bovine serum, and 10 mM HEPES buffer solution (pH 7.4).

### 3. Preparation of siRNA solution

siRNA was purchased from Proligo Japan as a freeze-dried product after the annealing of double strands. This siRNA was dissolved in DEPC-treated water (Nacalai Tesque) to adjust its concentration to 1 mM. The siRNA used was as follows: 1 mM Negative Control siRNA (21-mer sense strand; 5'-CAAGCUGACCCUGAAGUUCAU-3'; SEQ ID NO: 4 and 21-mer antisense strand; 5'-GAACUUCAGGGUCAGCUUGUU-3'; SEQ ID NO: 5, Proligo Japan) and 1 mM GAPDH siRNA (21-mer sense strand; 5'-CCACGAGAAAUAUGACAACUC-3; SEQ ID NO: 6 and 21-mer antisense strand; 5'-GUUGUCAUAUUUCUCGUGGUU-3'; SEQ ID NO: 7).

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 500000 DO11.10 cells were collected and centrifuged at 2000 rpm for 5 minutes with a high-speed tabletop centrifuge (Tomy Seiko) to remove the resulting culture supernatant. To the tube, 10 µl of 1 mM siRNA solution and 190 µl of the hypertonic solution were added. Following incubation at 37°C for 15 minutes, 1.5 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI). For repetitive infusion, the cells were collected every 24 hours of infusion, and reinfusion was performed under the same conditions.

### 19-2) Measurement of effect on siRNA infusion

The effect of types of oligosaccharide and polyhydric alcohol contained in the hypertonic solution on siRNA infusion into cells was examined with the efficiency of GAPDH siRNA-mediated knockdown of endogenous GAPDH mRNA as an indicator.

Total RNA was collected from each of the cells subjected to single infusion or repetitive infusion after 24 hours, 48 hours and 72 hours of infusion using an RNeasy kit (Qiagen). Primers specific to GAPDH mRNA (Forward Primer: 5'-TCACCACCATGGAGAAGGC-3' (SEQ ID NO: 15) and Reverse Primer: 5'-CTAAGCAGTTGGTGGTGCAG-3' (SEQ ID NO: 16)) and primers specific to cyclophilin mRNA that served as a normalization control (Forward Primer: 5'-GTCAACCCCACCGTGTTCT-3' (SEQ ID NO: 17) and Reverse Primer: 5'-GAGGTACCTGCTGTCTTTGG-3' (SEQ ID NO: 18)) were used to perform quantitative PCR in the same way as Example 2. The rate of decrease in GAPDH mRNA was calculated with the expression level of cyclophilin regarded as being equal between Negative Control siRNA-and GAPDH siRNA-infused groups. The result is shown in FIG. 9.

As can be seen from the result, in the group subjected to GAPDH siRNA single infusion, GAPDH mRNA disappeared 72.3% after 24 hours of infusion, 48.4% after 48 hours of infusion and 28.4% after 72 hours of infusion as compared with the Negative Control siRNA-infused group, whereas in the group subjected to GAPDH siRNA repetitive infusion, GAPDH mRNA disappeared 71.4% after 24 hours of infusion, 70.4% after 48 hours of infusion and 68.0% after 72 hours of infusion as compared with the Negative Control siRNA-infused group.

These results have shown that the method of infusion of the present invention can also efficiently infuse siRNA into murine hybridoma DO11.10 cells as with FITC-labeled oligonucleotide and that the infused siRNA exhibits knockdown activity. These results have also shown that reduction in the effect of knockdown with the passage of time was observed in single infusion, while the effect of siRNA-mediated knockdown can be maintained for any period by performing repetitive infusion.

### Example 20

### Infusion of siRNA into primary cultured differentiated rat adipocyte by method of nucleic acid infusion of the present invention

### 20-1) Infusion of siRNA into primary cultured differentiated rat adipocyte by method of nucleic acid infusion of the present invention

### 1. Preparation of cell

A primary cultured rat white adipocyte precursor cell was purchased from Hokudo. Specifically, the cells were received with them seeded at a density of 100000 cell/well in a 24-well plate (Nalge Nunc). Immediately after the receipt of the cells, the original medium was replaced with an accompanying differentiation medium, and the cells were cultured to obtain differentiated white adipocytes.

### 2. Preparation of hypertonic solution

A hypertonic solution was prepared to have the following composition: 1.0 M sucrose, 100 mM PEG1000, RPMI1640 (Invitrogen), 5% (v/v) fetal bovine serum, and 10 mM HEPES buffer solution (pH 7.4).

### 3. Preparation of siRNA solution

siRNA was purchased from Proligo Japan as a freeze-dried product after the annealing of double strands. This siRNA was dissolved in DEPC-treated water (Nacalai Tesque) to adjust its concentration to 1 mM. The siRNA used was as follows: 1 mM Negative Control siRNA (21-mer sense strand; 5'-CAAGCUGACCCUGAAGUUCAU-3'; SEQ ID NO: 4 and 21-mer antisense strand; 5'-GAACUUCAGGGUCAGCUUGUU-3'; SEQ ID NO: 5, Proligo Japan) and 1 mM GAPDH siRNA (21-mer sense strand; 5'-CCACGAGAAAUAUGACAACUC-3; SEQ ID NO: 6 and 21-mer antisense strand; 5'-GUUGUCAUAUUUCUCGUGGUU-3'; SEQ ID NO: 7).

### 3. Preparation of hypotonic solution

A hypotonic solution was prepared to be a mixture solution of a RPMI1640 medium and DEPC-treated water (6:4).

### 4. Growth medium

A medium included with a culture kit manufactured by Hokudo was used.

### 5. Infusion in 24-well plate

The culture supernatant of the differentiated adipocytes was removed, and 200 µl of the hypertonic solution containing siRNA was added to the cells. Following incubation at room temperature for 10 minutes, the hypertonic solution containing siRNA was removed. After the addition of 3 ml of the hypotonic solution and incubation at room temperature for 10 minutes, the hypotonic solution was removed, and 3 ml of the growth medium was added.

### 20-2) Measurement of effect on siRNA infusion

The effect of types of oligosaccharide and polyhydric alcohol contained in the hypertonic solution on siRNA infusion into cells was examined with the efficiency of GAPDH siRNA-mediated knockdown of endogenous GAPDH mRNA as an indicator.

Total RNA was collected from each of the cells subjected to single infusion after 24 hours, 48 hours and 72 hours of infusion using an RNeasy kit (Qiagen). Primers specific to GAPDH mRNA (Forward Primer: 5'-TCACCACCATGGAGAAGGC-3' (SEQ ID NO: 15) and Reverse Primer: 5'-CTAAGCAGTTGGTGGTGCAG-3' (SEQ ID NO: 16)) and primers specific to cyclophilin mRNA that served as a normalization control (Forward Primer: 5'-GTCAACCCCACCGTGTTCT-3' (SEQ ID NO: 17) and Reverse Primer: 5'-GAGGTACCTGCTGTCTTTGG-3' (SEQ ID NO: 18)) were used to perform quantitative PCR in the same way as Example 2. The rate of decrease in GAPDH mRNA was calculated with the expression level of cyclophilin regarded as being equal between Negative Control siRNA-and GAPDH siRNA-infused groups. The result is shown in FIG. 10.

As can be seen from the result, in the GAPDH siRNA-infused group, GAPDH mRNA disappeared 71.7% after 24 hours of infusion, 56.6% after 48 hours of infusion and 24.3% after 72 hours of infusion as compared with the Negative Control siRNA-infused group.

These results have shown that the method of infusion of the present invention can also efficiently infuse siRNA into primary cultured differentiated adipocytes as with FITC-labeled oligonucleotide and that the infused siRNA exhibits knockdown activity.

### Example 21

### Repetitive infusion of siRNA into murine macrophage-like cell line RAW264.7 cell using method of nucleic acid infusion of the resent invention

Murine macrophage-like cell line RAW264.7 cells were used to examine the effect of TLR2 siRNA repetitive infusion at a protein level.

### 21-1) Infusion of siRNA into murine macrophage-like cell line RAW264.7 cell by method of nucleic acid infusion of the present invention

### 1. Preparation of cell

RAW264.7 cells were cultured in RPMI1640 (Invitrogen) containing 10% (v/v) fetal calf serum in a T-75 flask (IWAKI) under conditions of 37°C and 5% CO₂.

### 2. Preparation of hypertonic solution

A hypertonic solution was prepared to have the following composition: 1.4 M sucrose, 100 mM PEG1000, RPMI1640 (Invitrogen), 5% (v/v) fetal bovine serum, and 10 mM HEPES buffer solution (pH 7.4).

### 3. Preparation of siRNA solution

siRNA was purchased from Proligo Japan as a freeze-dried product after the annealing of double strands. This siRNA was dissolved in DEPC-treated water (Nacalai Tesque) to adjust its concentration to 1 mM. The siRNA used was as follows: 1 mM Negative Control siRNA (21-mer sense strand; 5'-CAAGCUGACCCUGAAGUUCAU-3'; SEQ ID NO: 4 and 21-mer antisense strand; 5'-GAACUUCAGGGUCAGCUUGUU-3'; SEQ ID NO: 5, Proligo Japan) and 1 mM TLR2 siRNA (21-mer sense strand; 5'-GUCUAAAGUCGAUCCGCGACA-3; SEQ ID NO: 9 and 21-mer antisense strand; 5'-UCGCGGAUCGACUUUAGACUU-3'; SEQ ID NO: 10).

### 4. Hypotonic solution

Otsuka distilled water (Otsuka Pharmaceutical) was used as a hypotonic solution.

### 5. Growth medium

RPMI1640 (Invitrogen) was supplemented with 10% (v/v) fetal bovine serum and 60 mg/L kanamycin sulfate to prepare a growth medium.

### 6. Infusion in state of suspension

Into each 2.0-ml tube, 500000 RAW264.7 cells were collected and centrifuged at 2000 rpm for 5 minutes with a high-speed tabletop centrifuge (Tomy Seiko) to remove the resulting culture supernatant. To the tube, 10 µl of 1 mM siRNA solution and 190 µl of the hypertonic solution were added. Following incubation at 37°C for 10 minutes, 1.1 ml of Otsuka distilled water (Otsuka Pharmaceutical) was added. Following incubation for 10 minutes, the resulting mixture was centrifuged at 2000 rpm for 5 minutes with a high-speed tabletop centrifuge (Tomy Seiko) to remove the resulting culture supernatant. The mixture was resuspended in 2 ml of the growth medium, which was then seeded to a 6-well plate (IWAKI). The cells were collected after 24 hours of initial infusion, and reinfusion was performed under the same conditions.

### 21-2) Measurement at protein level

The effect of TLR2 siRNA infusion on endogenous TLR2 protein knockdown was examined.

Whole cell lysate was prepared from each of the Negative Control siRNA- and TLR2 siRNA-infused cells after 48 hours of initial infusion using 1% (w/v) SDS aqueous solution. Protein quantification was performed with a BCA kit (Pierce). After 40 µg of each sample was mixed with 1/4 volumes of 5x sample buffer, the resulting mixture was subjected to electrophoresis with 4%/20% gradient gel PAG Mini "Daiichi" 4/20 (Daiichi Pure Chemicals). After electrophoresis, BioRad Western Blotting System (BioRad) was used to blot the protein to Immobilon PVDF Transfer Membrane (Millipore). The membrane was then dipped in BlockAce (Dainippon Pharmaceutical) and blocked by overnight incubation at 4°C.

For TLR2 detection, the membrane was incubated overnight at 4°C in a solution of an anti-TLR2 Antibody (IMG-526, IMGENEX) diluted 1/250 (diluted with BlockAce), and then washed with TBST (Tris-buffered saline with 0.1% Tween 20). Subsequently, the membrane was incubated at room temperature for 2 hours in a solution of a goat anti-rabbit IgG-AP conjugate (BIOSOURCE) diluted 1/1000, and then washed with TBST (Tris-buffered saline with 0.1% Tween 20), followed by the staining of the membrane with Alkaline Phosphatase Conjugate Substrate Kit (BioRad). For GAPDH detection, the membrane was incubated overnight at 4°C in a solution of anti-GAPDH (V-18, SantaCruz) diluted 1/100, and then washed with TBST (Tris-buffered saline with 0.1% Tween 20). Subsequently, the membrane was incubated at room temperature for 2 hours in a solution of a donky anti-goat IgG-AP conjugate (CHEMICON) diluted 1/1000, and then washed with TBST (Tris-buffered saline with 0.1% Tween 20), followed by the staining of the membrane with Alkaline Phosphatase Conjugate Substrate Kit (BioRad). The result is shown in FIG. 11.

As can be seen from the result, in the TLR2 siRNA-infused group, the TLR2 protein decreased as compared with the Negative Control siRNA-infused group.

These results have shown that the effect of knockdown mediated by siRNA infused by the method of infusion of the present invention is observed at a protein level.

The results obtained in the above-described Examples 1 to 21 have revealed the followings:
1) the method of nucleic acid infusion of the present invention has the higher efficiency of nucleic acid infusion than those of conventional methods;
2) unlike conventional methods, the method of nucleic acid infusion of the present invention allows nucleic acid infusion without causing the morphological change and damage of cells, and the infused nucleic acid effectively functions in the cell;
3) the method of nucleic acid infusion of the present invention allows efficient nucleic acid infusion into cells that are previously reported to be hardly infused with a nucleic acid (such as a macrophage-like cell line RAW264.7 cell, a murine hybridoma DO11.10 cell, a primary cultured hepatocyte, a primary cultured myoblast, a primary cultured muscle cell, a primary cultured adipocyte precursor cell, a primary cultured adipocyte and a primary cultured neuron), and the method can be applied to any cell type without limitations;
4) the method of nucleic acid infusion of the present invention can be used regardless of the presence or absence of the electric charge of a nucleic acid;
5) ingredients in the hypertonic solution used in the method of nucleic acid infusion of the present invention include oligosaccharide and polyhydric alcohol, and specifically, any type of polyethylene glycol, glycerol, sucrose and sugar alcohol (such as mannitol, sorbitol, xylitol) can be used;
6) preferred molarity of the whole solute in the hypertonic solution used in the method of nucleic acid infusion of the present invention is approximately 0.5 to 2.1 M; and
7) the method of nucleic acid infusion of the present invention can be used in repetitive infusion.

### INDUSTRIAL APPLICABILITY

A novel method of nucleic acid infusion of the present invention allows nucleic acid infusion with higher efficiency than ever. In addition, the method of nucleic acid infusion of the present invention allows nucleic acid infusion without causing the morphological change and damage of cells. Therefore, the infused nucleic acid can effectively function in the cell. The method of infusion of the present invention also allows efficient nucleic acid infusion into cells that are previously reported to be hardly infused with a nucleic acid. Thus, the method of nucleic acid infusion of the present invention can be used quite effectively in gene functional analysis (e.g., gene functional analysis such as identification of a receptor-ligand pair) that targets every cell that is conventionally unable to be analyzed.

### Sequence Listing Free Text

SEQ ID NO: 1 is a base sequence of synthetic oligonucleotide.

SEQ ID NO: 2 is a base sequence of synthetic oligonucleotide.

SEQ ID NO: 3 is a base sequence of synthetic oligonucleotide.

SEQ ID NO: 4 is a base sequence of synthetic oligonucleotide.

SEQ ID NO: 5 is a base sequence of synthetic oligonucleotide.

SEQ ID NO: 6 is a base sequence of synthetic oligonucleotide.

SEQ ID NO: 7 is a base sequence of synthetic oligonucleotide.

SEQ ID NO: 8 is a base sequence of synthetic oligonucleotide.

SEQ ID NO: 9 is a base sequence of synthetic oligonucleotide.

SEQ ID NO: 10 is a base sequence of synthetic oligonucleotide.

SEQ ID NO: 11 is a base sequence of a PCR primer.

SEQ ID NO: 12 is a base sequence of a PCR primer.

SEQ ID NO: 13 is a base sequence of a PCR primer.

SEQ ID NO: 14 is a base sequence of a PCR primer.

SEQ ID NO: 15 is a base sequence of a PCR primer.

SEQ ID NO: 16 is a base sequence of a PCR primer.

SEQ ID NO: 17 is a base sequence of a PCR primer.

SEQ ID NO: 18 is a base sequence of a PCR primer.

## Claims

1. A method of nucleic acid infusion, comprising the following steps (a) and (b):
(a) bringing a nucleic acid, a hypertonic solution and cells into contact with each other; and
(b) lowering osmotic pressure of the hypertonic solution after the step (a).

2. The method of nucleic acid infusion according to claim 1, wherein the step (b) comprises lowering the osmotic pressure by bringing a hypotonic solution and the cells into contact with each other.

3. The method of nucleic acid infusion according to claim 1 or 2, wherein the nucleic acid is oligonucleotide.

4. The method of nucleic acid infusion according to claim 3, wherein the oligonucleotide is single-stranded oligonucleotide, double-stranded oligonucleotide or an analog thereof.

5. The method of nucleic acid infusion according to claim 3 or 4, wherein the oligonucleotide is deoxyribonucleotide (DNA), ribonucleotide (RNA), phosphorothioate oligodeoxynucleotide, a 2'-O-(2-methoxy)ethyl-modified nucleic acid (2'-MOE-modified nucleic acid), small interfering RNA (siRNA), a locked nucleic acid (LNA), a peptide nucleic acid (PNA) or morpholino antisense oligonucleotide.

6. The method of nucleic acid infusion according to any of claims 1 to 5, wherein the hypertonic solution comprises at least one substance which is oligosaccharide or polyhydric alcohol.

7. The method of nucleic acid infusion according to claim 6, wherein the oligosaccharide is disaccharide.

8. The method of nucleic acid infusion according to claim 7, wherein the disaccharide is sucrose, maltose or lactose.

9. The method of nucleic acid infusion according to claim 6, wherein the polyhydric alcohol is diol, triol, polyol or sugar alcohol.

10. The method of nucleic acid infusion according to claim 9, wherein the diol is a glycol derivative.

11. The method of nucleic acid infusion according to claim 9, wherein the triol is a glycerol derivative.

12. The method of nucleic acid infusion according to claim 9, wherein the polyol is a polyglycol derivative.

13. The method of nucleic acid infusion according to claim 9, wherein the sugar alcohol is monosaccharide alcohol or oligosaccharide alcohol.

14. The method of nucleic acid infusion according to claim 13, wherein the oligosaccharide alcohol is disaccharide alcohol.

15. The method of nucleic acid infusion according to claim 10, wherein the glycol derivative is ethylene glycol.

16. The method of nucleic acid infusion according to claim 11, wherein the glycerol derivative is glycerol.

17. The method of nucleic acid infusion according to claim 12, wherein the polyglycol derivative is polyethylene glycol.

18. The method of nucleic acid infusion according to claim 17, wherein the polyethylene glycol has a molecular weight of 2000 or less.

19. The method of nucleic acid infusion according to claim 18, wherein the polyethylene glycol is PEG200, PEG300, PEG400, PEG600, PEG1000, PEG1500, PEG1540 or PEG 2000.

20. The method of nucleic acid infusion according to claim 13, wherein the monosaccharide alcohol is mannitol, sorbitol, xylitol or erythritol.

21. The method of nucleic acid infusion according to claim 14, wherein the disaccharide alcohol is maltitol, lactitol or reduced palatinose.

22. The method of nucleic acid infusion according to claim 6, wherein the hypertonic solution comprises (1) at least one substance which is oligosaccharide or sugar alcohol and (2) at least one substance which is diol, triol or polyol.

23. The method of nucleic acid infusion according to claim 22, wherein the oligosaccharide is disaccharide.

24. The method of nucleic acid infusion according to claim 23, wherein the disaccharide is sucrose, maltose or lactose.

25. The method of nucleic acid infusion according to claim 22, wherein the sugar alcohol is monosaccharide alcohol or oligosaccharide alcohol.

26. The method of nucleic acid infusion according to claim 25, wherein the oligosaccharide alcohol is disaccharide alcohol.

27. The method of nucleic acid infusion according to claim 25, wherein the monosaccharide alcohol is mannitol, sorbitol, xylitol or erythritol.

28. The method of nucleic acid infusion according to claim 26, wherein the disaccharide alcohol is maltitol, lactitol or reduced palatinose.

29. The method of nucleic acid infusion according to claim 22, wherein the diol is a glycol derivative.

30. The method of nucleic acid infusion according to claim 22, wherein the triol is a glycerol derivative.

31. The method of nucleic acid infusion according to claim 22, wherein the polyol is a polyglycol derivative.

32. The method of nucleic acid infusion according to claim 29, wherein the glycol derivative is ethylene glycol.

33. The method of nucleic acid infusion according to claim 30, wherein the glycerol derivative is glycerol.

34. The method of nucleic acid infusion according to claim 31, wherein the polyglycol derivative is polyethylene glycol.

35. The method of nucleic acid infusion according to claim 34, wherein the polyethylene glycol has a molecular weight of 2000 or less.

36. The method of nucleic acid infusion according to claim 35, wherein the polyethylene glycol is PEG200, PEG300, PEG400, PEG600, PEG1000, PEG1500, PEG1540 or PEG2000.

37. The method of nucleic acid infusion according to claim 22, wherein the hypertonic solution comprises (1) at least one substance selected from sucrose, mannitol, sorbitol and xylitol and (2) at least one substance selected from PEG200, PEG300, PEG400, PEG600, PEG1000, PEG1500, PEG1540, PEG2000 and glycerol.

38. The method of nucleic acid infusion according to claim 37, wherein the hypertonic solution comprises (1) sucrose, mannitol, sorbitol or xylitol and (2) PEG200, PEG300, PEG400, PEG600, PEG1000, PEG1500, PEG1540, PEG2000 or glycerol.

39. The method of nucleic acid infusion according to claim 6, wherein the hypertonic solution comprises one substance which is oligosaccharide or sugar alcohol.

40. The method of nucleic acid infusion according to claim 39, wherein the oligosaccharide is disaccharide.

41. The method of nucleic acid infusion according to claim 40, wherein the disaccharide is sucrose, maltose or lactose.

42. The method of nucleic acid infusion according to claim 39, wherein the sugar alcohol is monosaccharide alcohol or oligosaccharide alcohol.

43. The method of nucleic acid infusion according to claim 42, wherein the oligosaccharide alcohol is disaccharide alcohol.

44. The method of nucleic acid infusion according to claim 42, wherein the monosaccharide alcohol is mannitol, sorbitol, xylitol or erythritol.

45. The method of nucleic acid infusion according to claim 43, wherein the disaccharide alcohol is maltitol, lactitol or reduced palatinose.

46. The method of nucleic acid infusion according to claim 39, wherein the hypertonic solution comprises sucrose, mannitol, sorbitol, xylitol or maltitol.

47. The method of nucleic acid infusion according to any of claims 1 to 46, wherein the oligosaccharide and/or the polyhydric alcohol in the hypertonic solution has a total molarity of 0.29 M to 3 M.

48. The method of nucleic acid infusion according to claim 47, wherein the oligosaccharide and/or the polyhydric alcohol in the hypertonic solution has a total molarity of 0.5 M to 2.1 M.

49. The method of nucleic acid infusion according to any of claims 2 to 48, wherein the hypotonic solution has isotonic osmotic pressure or lower.

50. A reagent for nucleic acid infusion, comprising as an ingredient at least one substance which is oligosaccharide or polyhydric alcohol.

51. The reagent for nucleic acid infusion according to claim 50, wherein the reagent is used in a method of nucleic acid infusion according to any of claims 1 to 49.

52. The reagent for nucleic acid infusion according to claim 50 or 51, wherein the oligosaccharide is disaccharide.

53. The reagent for nucleic acid infusion according to claim 52, wherein the disaccharide is sucrose, maltose or lactose.

54. The reagent for nucleic acid infusion according to claim 50 or 51, wherein the polyhydric alcohol is diol, triol, polyol or sugar alcohol.

55. The reagent for nucleic acid infusion according to claim 54, wherein the diol is a glycol derivative.

56. The reagent for nucleic acid infusion according to claim 54, wherein the triol is a glycerol derivative.

57. The reagent for nucleic acid infusion according to claim 54, wherein the polyol is a polyglycol derivative.

58. The reagent for nucleic acid infusion according to claim 54, wherein the sugar alcohol is monosaccharide alcohol or oligosaccharide alcohol.

59. The reagent for nucleic acid infusion according to claim 58, wherein the oligosaccharide alcohol is disaccharide alcohol.

60. The reagent for nucleic acid infusion according to claim 55, wherein the glycol derivative is ethylene glycol.

61. The reagent for nucleic acid infusion according to claim 56, wherein the glycerol derivative is glycerol.

62. The reagent for nucleic acid infusion according to claim 57, wherein the polyglycol derivative is polyethylene glycol.

63. The reagent for nucleic acid infusion according to claim 62, wherein the polyethylene glycol has a molecular weight of 2000 or less.

64. The reagent for nucleic acid infusion according to claim 63, wherein the polyethylene glycol is PEG200, PEG300, PEG400, PEG600, PEG1000, PEG1500, PEG1540 or PEG2000.

65. The reagent for nucleic acid infusion according to claim 58, wherein the monosaccharide alcohol is mannitol, sorbitol, xylitol or erythritol.

66. The reagent for nucleic acid infusion according to claim 59, wherein the disaccharide alcohol is maltitol, lactitol or reduced palatinose.

67. The reagent for nucleic acid infusion according to claim 50 or 51, wherein the reagent comprises as ingredients (1) at least one substance which is oligosaccharide or sugar alcohol and (2) at least one substance which is diol, triol or polyol.

68. The reagent for nucleic acid infusion according to claim 67, wherein the oligosaccharide is disaccharide.

69. The reagent for nucleic acid infusion according to claim 68, wherein the disaccharide is sucrose, maltose or lactose.

70. The reagent for nucleic acid infusion according to claim 67, wherein the sugar alcohol is monosaccharide alcohol or oligosaccharide alcohol.

71. The reagent for nucleic acid infusion according to claim 70, wherein the oligosaccharide alcohol is disaccharide alcohol.

72. The reagent for nucleic acid infusion according to claim 70, wherein the monosaccharide alcohol is mannitol, sorbitol, xylitol or erythritol.

73. The reagent for nucleic acid infusion according to claim 71, wherein the disaccharide alcohol is maltitol, lactitol or reduced palatinose.

74. The reagent for nucleic acid infusion according to claim 67, wherein the diol is a glycol derivative.

75. The reagent for nucleic acid infusion according to claim 67, wherein the triol is a glycerol derivative.

76. The reagent for nucleic acid infusion according to claim 67, wherein the polyol is a polyglycol derivative.

77. The reagent for nucleic acid infusion according to claim 74, wherein the glycol derivative is ethylene glycol.

78. The reagent for nucleic acid infusion according to claim 75, wherein the glycerol derivative is glycerol.

79. The reagent for nucleic acid infusion according to claim 76, wherein the polyglycol derivative is polyethylene glycol.

80. The reagent for nucleic acid infusion according to claim 79, wherein the polyethylene glycol has a molecular weight of 2000 or less.

81. The reagent for nucleic acid infusion according to claim 80, wherein the polyethylene glycol is PEG200, PEG300, PEG400, PEG600, PEG1000, PEG1500, PEG1540 or PEG2000.

82. The reagent for nucleic acid infusion according to claim 67, wherein the reagent comprises as ingredients (1) at least one substance selected from sucrose, mannitol, sorbitol and xylitol and (2) at least one substance selected from PEG200, PEG300, PEG400, PEG600, PEG1000, PEG1500, PEG1540, PEG2000 and glycerol.

83. The reagent for nucleic acid infusion according to claim 82, wherein the reagent comprises as ingredients (1) sucrose, mannitol, sorbitol or xylitol and (2) PEG200, PEG300, PEG400, PEG600, PEG1000, PEG1500, PEG1540, PEG2000 or glycerol.

84. The reagent for nucleic acid infusion according to claim 50 or 51, wherein the reagent comprises as an ingredient at least one substance which is oligosaccharide or sugar alcohol.

85. The method for nucleic acid infusion according to claim 84, wherein the oligosaccharide is disaccharide.

86. The reagent for nucleic acid infusion according to claim 85, wherein the disaccharide is sucrose, maltose or lactose.

87. The reagent for nucleic acid infusion according to claim 84, wherein the sugar alcohol is monosaccharide alcohol or oligosaccharide alcohol.

88. The reagent for nucleic acid infusion according to claim 87, wherein the oligosaccharide alcohol is disaccharide alcohol.

89. The reagent for nucleic acid infusion according to claim 87, wherein the monosaccharide alcohol is mannitol, sorbitol, xylitol or erythritol.

90. The reagent for nucleic acid infusion according to claim 88, wherein the disaccharide alcohol is maltitol, lactitol or reduced palatinose.

91. The reagent for nucleic acid infusion according to claim 84, wherein the reagent comprises as an ingredient sucrose, mannitol, sorbitol, xylitol or maltitol.

92. The reagent for nucleic acid infusion according to any of claims 50 to 91, wherein the ingredient is in the form of a solid or liquid.

93. The reagent for nucleic acid infusion according to claim 92, wherein the reagent comprises two or more ingredients which are each oligosaccharide or polyhydric alcohol, and the ingredients are each independently in the form of a solid or liquid.

94. The reagent for nucleic acid infusion according to any of claims 50 to 93, wherein the reagent is prepared in advance so that the oligosaccharide and/or the polyhydric alcohol in a hypertonic solution has a total molarity of 0.29 M to 3 M.

95. The reagent for nucleic acid infusion according to claim 94, wherein the reagent is prepared in advance so that the oligosaccharide and/or the polyhydric alcohol in a hypertonic solution has a total molarity of 0.5 M to 2.1 M.

96. A kit for nucleic acid infusion, comprising a reagent according to any of claims 50 to 95.

97. Use of a reagent or a kit according to any of claims 50 to 96 in nucleic acid infusion.

98. A nucleic acid infusion agent, comprising as an ingredient a reagent or a kit according to any of claims 50 to 96.

99. A cell with a nucleic acid infused thereinto by a method of infusion according to any of claims 1 to 49.

100. A macrophage-like cell line RAW264.7 cell, a hybridoma DO11.10 cell, a primary cultured hepatocyte, a primary cultured myoblast, a primary cultured muscle cell, a primary cultured adipocyte precursor cell, a primary cultured adipocyte or a primary cultured neuron with a nucleic acid infused thereinto by a method of infusion according to any of claims 1 to 49.

101. Cell-derived RNA or protein prepared from a cell according to claim 99 or 100.

102. A method of functional analysis of a gene or protein associated with an infused nucleic acid, **characterized by** using a cell according to claim 99 or 100 or RNA or protein according to claim 101.
